# FASCICULE DE BREVET EUROPEEN

(11) **EP 2 582 810 B1**
(45) Date de publication et mention de la délivrance du brevet: **25.05.2016**
(21) Numéro de dépôt: 11736126.1
(22) Date de dépôt: 16.06.2011
(51) Int. Cl.: C12N 9/88, C12P 19/04, C12P 19/12, C12N 1/20, C12R 1/01

(54) **ULVANE LYASE, PROCEDE DE FABRICATION ET UTILISATIONS**
ULVANLYASE, HERSTELLUNGSVERFAHREN DAFÜR UND VERWENDUNG
ULVAN LYASE, METHOD FOR MANUFACTURING SAME, AND USES THEREOF

(30) Priorité: 18.06.2010 FR 1002588
(43) Date de publication de la demande: 24.04.2013
(73) Titulaire: Centre National de la Recherche Scientifique (CNRS), 75794 Paris Cedex 16 (FR); Centre d'Etude et de Valorisation des Algues-CEVA, 22610 Pleubian (FR); Université Pierre et Marie Curie, 75005 Paris (FR)
(72) Inventeur: HELBERT, William, F-29680 Roscoff (FR); NYVALL-COLLEN, Pi, F-29680 Roscoff (FR); LERAT, Yannick, 22620 Ploubazlanec (FR); SASSI, Jean-François, 22620 Ploubazlanec (FR)
(74) Mandataire: Novagraaf Technologies
(86) Numéro de dépôt international: PCT/FR2011/051384
(87) Numéro de publication internationale: WO 2011/157966

(56) Documents cités:
- WO-A2-2009/016275
- LAHAYE M ET AL: "Fine chemical structure analysis of oligosaccharides produced by an ulvan-lyase degradation of the water-soluble cell-wall polysaccharides from Ulva sp. (Ulvales, Chlorophyta)", CARBOHYDRATE RESEARCH, PERGAMON, GB, vol. 304, no. 3-4, 28 novembre 1997 (1997-11-28), pages 325-333, XP004101618, ISSN: 0008-6215, DOI: DOI:10.1016/S0008-6215(97)00270-X
- DONG EUN KIM ET AL: "Cloning and Characterization of Alginate Lyase from a Marine Bacterium Streptomyces sp. ALG-5", MARINE BIOTECHNOLOGY, SPRINGER-VERLAG, NE, vol. 11, no. 1, 14 juin 2008 (2008-06-14), pages 10-16, XP019663865, ISSN: 1436-2236
- SHIMIZU E ET AL: "cDNA cloning of an alginate lyase from abalone, Haliotis discus hannai", CARBOHYDRATE RESEARCH, PERGAMON, GB, vol. 338, no. 24, 21 novembre 2003 (2003-11-21), pages 2841-2852, XP004479502, ISSN: 0008-6215, DOI: DOI:10.1016/J.CARRES.2003.08.009
- ZAHURA U A ET AL: "An endo-beta-1,4-mannanase, AkMan, from the common sea hare Aplysia kurodai", COMPARATIVE BIOCHEMISTRY AND PHYSIOLOGY. PART B, BIOCHEMISTRYAND MOLECULAR BIOLOGY, ELSEVIER,OXFORD, GB, vol. 157, no. 1, septembre 2010 (2010-09), pages 137-143, XP027112730, ISSN: 1096-4959 [extrait le 2010-06-04]
- BARBEYRON TRISTAN ET AL: "Persicivirga ulvanivorans sp nov., a marine member of the family Flavobacteriaceae that degrades ulvan from green algae", INTERNATIONAL JOURNAL OF SYSTEMATIC AND EVOLUTIONARY MICROBIOLOGY, vol. 61, no. Part 8, août 2011 (2011-08), pages 1899-1905, XP009153107,

### SEQUENCE LISTING

<110> Centre National de la Recherche Scientifique (CNRS) Centre d'Etude et de valorisation des Algues Université Pierre et Marie Curie
<120> ULVANE LYASE, PROCEDE DE FABRICATION ET UTILISATIONS
<130> BIP206420FR00
<150> FR 1002588
   <151> 2010-06-18
<160> 70
<170> PatentIn version 3.5
<210> 1
   <211> 298
   <212> PRT
   <213> Artificial Sequence
<220>
   <223> CNCM n° I-4324
<400> 1
   Ala Pro Asp Glu Asp Thr Asn Ser Ser Ile Ala Cys Pro Ser Ser Gly
   1               5                   10                  15
   Val Phe Gln Asn Asn Thr Thr Arg Asp Val Asp Ile Ala Asn Pro Asp
               20                  25                  30
   Asn Val Gly Thr Val Asp Asp Arg Thr Cys Tyr Ala Asp Tyr Tyr Glu
           35                  40                  45
   Thr Ser Val Tyr Gly Glu Thr Trp Gly Ala Tyr Asn Ile Thr Phe Asn
       50                  55                  60
   Ser Asn His Trp Asp Ala Pro Asn Thr Leu Gln Pro Arg Ile Glu Arg
   65                  70                  75                  80
   Ser Leu Ser Arg Ser Gln Glu Thr Gly Val Gly Ser Tyr Ala Arg Phe
                   85                  90                  95
   Thr Gly Thr Leu Arg Ile Leu Glu Val Gly Asn Thr Gly Thr Phe Gly
               100                 105                 110
   Ser Thr Gly Ser Tyr Leu Met Gln Ala Lys Gly Lys His Thr Gly Gly
           115                 120                 125
   Gly Gly Ser Asn Asp Pro Ala Ile Cys Leu Tyr Leu Ala Arg Pro Val
       130                 135                 140
   Tyr Gly Pro Asp Ala Asn Gly Asn Gln Val Gln Val Ser Phe Asp Ile
   145                 150                 155                 160
   Trp Arg Glu Gln Ile Asn Phe Arg Gly Gly Ser Gly Ala Ala Gly Arg
                   165                 170                 175
   Thr Glu Val Phe Leu Arg Asn Val Leu Lys Asp Glu Ile Ile Asp Ile
               180                 185                 190
   Glu Leu Glu Val Gly Phe Arg Gln Asp Pro Asn Asp Pro Asn Leu Lys
           195                 200                 205
   Ile His Tyr Ser Asp Ala Ile Ile Gly Gly Gln Val Phe Asn Trp Asn
       210                 215                 220
   Ile Pro Glu Pro Glu Arg Gly Arg Glu Ser Gly Ile Arg Tyr Gly Val
   225                 230                 235                 240
   Tyr Arg Val Lys Gly Gly Arg Ala Gln Met Arg Trp Ala Asn Thr Thr
                   245                 250                 255
   Tyr Gln Lys Val Glu Val Val Asp Asn Ser Thr Ile Pro Ala Ala Asp
               260                 265                 270
   Ile Tyr Arg Ile Lys Asn Val Glu Thr Gly Glu Tyr Leu Thr Ser Ser
           275                 280                 285
   Gly Ser Ser Ile Ile Ala Ser Thr Ser Gly
       290                 295
<210> 2
   <211> 189
   <212> PRT
   <213> Artificial Sequence
<220>
   <223> CNCM n° I-4324
<400> 2
   Thr Gly Ser Asp Lys Glu Trp Glu Ile Ile Ser Ala Gly Ser Gly Ser
   1               5                   10                  15
   Ser Tyr Val Asn Ile Asp Ser Gln Val Arg Gly Ile Ile Arg Phe Thr
               20                  25                  30
   Gly Gly Ser Ser Asn Pro Gly Leu Val Ser Thr Asn Phe Ser Pro Pro
           35                  40                  45
   Asn Thr Asp Thr Asp Lys Val Trp Thr Val Ile Asp Asn Asn Asp Gly
       50                  55                  60
   Thr Val Ser Phe Glu Thr Arg Asn Leu Gly Arg Phe Leu Tyr His Asp
   65                  70                  75                  80
   Thr Asn Asn Met Ile Thr His Ser Ala Asn Ile Asp Asp Arg Ser Lys
                   85                  90                  95
   Trp Asn Leu Glu Ser Thr Thr Leu Ser Val Asp Ser Gln Gln Ile Ala
               100                 105                 110
   Ser Val Gly Val Tyr Pro Asn Pro Thr Val Asp Gly Phe Thr Ile Ser
           115                 120                 125
   Leu Asp Asn Ile Ser Ala Glu Lys Val Gln Ile Phe Asn Leu Leu Gly
       130                 135                 140
   Met Leu Val Tyr Glu Gln Lys Thr Asn Glu Ser Ser Ile His Ile Asp
   145                 150                 155                 160
   Asn Met Asp Asn Phe Asp Ser Gly Met Tyr Ile Ile Ser Val Thr Ala
                   165                 170                 175
   Asn Asp Asn Lys Val Tyr Gln Thr Lys Leu Ile Val Asn
               180                 185
<210> 3
   <211> 47
   <212> PRT
   <213> Artificial Sequence
<220>
   <223> CNCM n° I-4324
<400> 3
   Met Val Phe Phe Lys Asp Leu Phe Ile Phe Lys Ser Leu Ile Lys Gly
   1 5 10 15
   Ser Leu Tyr Ser Gly His Met Lys Lys Lys Leu Leu Asn Tyr Leu Pro
   20 25 30
   Leu Phe Ala Leu Met Leu Phe Thr Val Ser Met Met Ala Gln Thr
   35 40 45
<210> 4
   <211> 534
   <212> PRT
   <213> Artificial Séquence
<220>
   <223> CNCM n° I-4324
<400> 4
   Met Val Phe Phe Lys Asp Leu Phe Ile Phe Lys Ser Leu Ile Lys Gly
   1 5 10 15
   Ser Leu Tyr Ser Gly His Met Lys Lys Lys Leu Leu Asn Tyr Leu Pro
   20 25 30
   Leu Phe Ala Leu Met Leu Phe Thr Val Ser Met Met Ala Gln Thr Ala
   35 40 45
   Pro Asp Glu Asp Thr Asn Ser Ser Ile Ala Cys Pro Ser Ser Gly Val
   50 55 60
   Phe Gln Asn Asn Thr Thr Arg Asp Val Asp Ile Ala Asn Pro Asp Asn
   65                  70                  75                  80
   Val Gly Thr Val Asp Asp Arg Thr Cys Tyr Ala Asp Tyr Tyr Glu Thr
                   85                  90                  95
   Ser Val Tyr Gly Glu Thr Trp Gly Ala Tyr Asn Ile Thr Phe Asn Ser
               100                 105                 110
   Asn His Trp Asp Ala Pro Asn Thr Leu Gln Pro Arg Ile Glu Arg Ser
           115                 120                 125
   Leu Ser Arg Ser Gln Glu Thr Gly Val Gly Ser Tyr Ala Arg Phe Thr
       130                 135                 140
   Gly Thr Leu Arg Ile Leu Glu Val Gly Asn Thr Gly Thr Phe Gly Ser
   145                 150                 155                 160
   Thr Gly Ser Tyr Leu Met Gln Ala Lys Gly Lys His Thr Gly Gly Gly
                   165                 170                 175
   Gly Ser Asn Asp Pro Ala Ile Cys Leu Tyr Leu Ala Arg Pro Val Tyr
               180                 185                 190
   Gly Pro Asp Ala Asn Gly Asn Gln Val Gln Val Ser Phe Asp Ile Trp
           195                 200                 205
   Arg Glu Gln Ile Asn Phe Arg Gly Gly Ser Gly Ala Ala Gly Arg Thr
       210                 215                 220
   Glu Val Phe Leu Arg Asn Val Leu Lys Asp Glu Ile Ile Asp Ile Glu
   225                 230                 235                 240
   Leu Glu Val Gly Phe Arg Gln Asp Pro Asn Asp Pro Asn Leu Lys Ile
                   245                 250                 255
   His Tyr Ser Asp Ala Ile Ile Gly Gly Gln Val Phe Asn Trp Asn Ile
               260                 265                 270
   Pro Glu Pro Glu Arg Gly Arg Glu Ser Gly Ile Arg Tyr Gly Val Tyr
           275                 280                 285
   Arg Val Lys Gly Gly Arg Ala Gln Met Arg Trp Ala Asn Thr Thr Tyr
       290                 295                 300
   Gln Lys Val Glu Val Val Asp Asn Ser Thr Ile Pro Ala Ala Asp Ile
   305                 310                 315                 320
   Tyr Arg Ile Lys Asn Val Glu Thr Gly Glu Tyr Leu Thr Ser Ser Gly
                   325                 330                 335
   Ser Ser Ile Ile Ala Ser Thr Ser Gly Thr Gly Ser Asp Lys Glu Trp
               340                 345                 350
   Glu Ile Ile Ser Ala Gly Ser Gly Ser Ser Tyr Val Asn Ile Asp Ser
           355                 360                 365
   Gln Val Arg Gly Ile Ile Arg Phe Thr Gly Gly Ser Ser Asn Pro Gly
       370                 375                 380
   Leu Val Ser Thr Asn Phe Ser Pro Pro Asn Thr Asp Thr Asp Lys Val
   385                 390                 395                 400
   Trp Thr Val Ile Asp Asn Asn Asp Gly Thr Val Ser Phe Glu Thr Arg
                   405                 410                 415
   Asn Leu Gly Arg Phe Leu Tyr His Asp Thr Asn Asn Met Ile Thr His
               420                 425                 430
   Ser Ala Asn Ile Asp Asp Arg Ser Lys Trp Asn Leu Glu Ser Thr Thr
           435                 440                 445
   Leu Ser Val Asp Ser Gln Gln Ile Ala Ser Val Gly Val Tyr Pro Asn
       450                 455                 460
   Pro Thr Val Asp Gly Phe Thr Ile Ser Leu Asp Asn Ile Ser Ala Glu
   465                 470                 475                 480
   Lys Val Gln Ile Phe Asn Leu Leu Gly Met Leu Val Tyr Glu Gln Lys
                   485                 490                 495
   Thr Asn Glu Ser Ser Ile His Ile Asp Asn Met Asp Asn Phe Asp Ser
               500                 505                 510
   Gly Met Tyr Ile Ile Ser Val Thr Ala Asn Asp Asn Lys Val Tyr Gln
           515                 520                 525
   Thr Lys Leu Ile Val Asn
       530
<210> 5
   <211> 896
   <212> DNA
   <213> Artificial Sequence
<220>
   <223> CNCM n° I-4324
<400> 5
   gcgcctgatg aggatacaaa ttctagtata gcttgtccta gctcaggtgt ttttcaaaat 60
   aatacgacta gagatgtaga tatagccaat cctgataatg tgggtactgt tgatgataga 120
   acctgttatg cagattatta tgaaactagt gtttatggag aaacttgggg agcatataac 180
   ataaccttta attctaatca ttgggatgca cctaacacat tacaacctag aatagagcga 240
   tcattatcaa ggtctcaaga aactggtgtg ggaagttatg cgcgattcac tgggacattg 300
   agaattcttg aagttggtaa taccggtact ttcggtagta ctggaagtta tctgatgcag 360
   gccaaaggta agcacacggg cggtggtgga tcaaatgatc cggcgatctg tttgtattta 420
   gcaagaccag tttatggacc tgacgctaat ggtaatcaag tacaggtatc atttgatatt 480
   tggagggaac agatcaattt tagaggtgga tccggagcag ctggaagaac cgaggtcttt 540
   cttagaaatg ttttaaaaga tgaaataatt gatatagaat tagaagtagg atttagacaa 600
   gatcctaatg atcctaattt aaaaatacac tattctgatg ctatcatagg tggtcaagta 660
   tttaattgga atattccgga accagaacga ggaagagaat ctggtatcag atatggggtt 720
   taccgtgtaa aaggaggaag agcacaaatg agatgggcaa atacgactta tcagaaagta 780
   gaagttgtag ataatagtac tatccctgca gcagatattt acaggataaa aaatgtagag 840
   actggagaat atttaacatc atcaggttca agcattatcg caagtacgtc aggaac 896
<210> 6
   <211> 569
   <212> DNA
   <213> Artificial Sequence
<220>
   <223> CNCM n° I-4324
<400> 6
   actggttcag ataaagaatg ggagataatc tcagctggat ctggctctag ctatgtcaat 60
   atcgatagtc aagttagagg aataataaga tttactggtg gatcgtcaaa tccaggatta 120
   gtaagtacaa atttttcacc gccaaataca gatacagata aagtatggac tgttattgat 180
   aataatgatg gaactgttag ttttgaaaca cgtaatctgg gtaggttttt atatcatgat 240
   accaataata tgataacaca ttcagctaat atagatgata gaagtaaatg gaatcttgaa 300
   tccactactt taagtgttga tagtcagcaa attgcttctg taggtgtgta tcctaaccct 360
   acggttgatg gctttacaat atccttagat aatattagtg ctgagaaagt tcaaattttc 420
   aaccttttag gaatgttggt atacgaacaa aagacaaatg agtcaagtat ccacatagat 480
   aacatggata actttgattc aggtatgtat atcattagtg tcaccgcaaa tgataacaag 540
   gtttatcaaa ccaagctcat tgtaaatta 569
<210> 7
   <211> 143
   <212> DNA
   <213> Artificial Sequence
<220>
   <223> CNCM n° I-4324
<400> 7
   atggtgtttt ttaaagattt attcatcttt aaatctttaa ttaaaggatc tttatattca      60
   ggacacatga aaaaaaaatt attgaattat ttaccattgt ttgcattgat gctatttaca     120
   gtgtcaatga tggctcaaac agc                                             143
<210> 8
   <211> 1607
   <212> DNA
   <213> Artificial Sequence
<220>
   <223> CNCM n° I-4324
<400> 8
   atggtgtttt ttaaagattt attcatcttt aaatctttaa ttaaaggatc tttatattca      60
   ggacacatga aaaaaaaatt attgaattat ttaccattgt ttgcattgat gctatttaca     120
   gtgtcaatga tggctcaaac agcgcgcctg atgaggatac aaattctagt atagcttgtc     180
   ctagctcagg tgtttttcaa aataatacga ctagagatgt agatatagcc aatcctgata     240
   atgtgggtac tgttgatgat agaacctgtt atgcagatta ttatgaaact agtgtttatg     300
   gagaaacttg gggagcatat aacataacct ttaattctaa tcattgggat gcacctaaca     360
   cattacaacc tagaatagag cgatcattat caaggtctca agaaactggt gtgggaagtt     420
   atgcgcgatt cactgggaca ttgagaattc ttgaagttgg taataccggt actttcggta     480
   gtactggaag ttatctgatg caggccaaag gtaagcacac gggcggtggt ggatcaaatg     540
   atccggcgat ctgtttgtat ttagcaagac cagtttatgg acctgacgct aatggtaatc     600
   aagtacaggt atcatttgat atttggaggg aacagatcaa ttttagaggt ggatccggag     660
   cagctggaag aaccgaggtc tttcttagaa atgttttaaa agatgaaata attgatatag     720
   aattagaagt aggatttaga caagatccta atgatcctaa tttaaaaata cactattctg     780
   atgctatcat aggtggtcaa gtatttaatt ggaatattcc ggaaccagaa cgaggaagag     840
   aatctggtat cagatatggg gtttaccgtg taaaaggagg aagagcacaa atgagatggg     900
   caaatacgac ttatcagaaa gtagaagttg tagataatag tactatccct gcagcagata     960
   tttacaggat aaaaaatgta gagactggag aatatttaac atcatcaggt tcaagcatta    1020
   tcgcaagtac gtcaggaact ggttcagata aagaatggga gataatctca gctggatctg    1080
   gctctagcta tgtcaatatc gatagtcaag ttagaggaat aataagattt actggtggat    1140
   cgtcaaatcc aggattagta agtacaaatt tttcaccgcc aaatacagat acagataaag    1200
   tatggactgt tattgataat aatgatggaa ctgttagttt tgaaacacgt aatctgggta    1260
   ggtttttata tcatgatacc aataatatga taacacattc agctaatata gatgatagaa    1320
   gtaaatggaa tcttgaatcc actactttaa gtgttgatag tcagcaaatt gcttctgtag    1380
   gtgtgtatcc taaccctacg gttgatggct ttacaatatc cttagataat attagtgctg    1440
   agaaagttca aattttcaac cttttaggaa tgttggtata cgaacaaaag acaaatgagt    1500
   caagtatcca catagataac atggataact ttgattcagg tatgtatatc attagtgtca    1560
   ccgcaaatga taacaaggtt tatcaaacca agctcattgt aaattag                  1607
<210> 9
   <211> 7
   <212> PRT
   <213> Artificial Sequence
<220>
   <223> CNCM n° I-4324
<400> 9
   Pro Asn Asp Pro Asn Leu Lys
   1               5
<210> 10
   <211> 15
   <212> PRT
   <213> Artificial Sequence
<220>
   <223> CNCM n° I-4324
<400> 10
   Leu Leu Glu Val Gly Asn Thr Gly Thr Phe Gly Ser Thr Gly Ser
   1               5                   10                  15
<210> 11
   <211> 8
   <212> PRT
   <213> Artificial Sequence
<220>
   <223> CNCM n° I-4324
<400> 11
   Asp Leu Ala Asn Pro Asp Asn Val
   1               5
<210> 12
   <211> 14
   <212> PRT
   <213> Artificial Sequence
<220>
   <223> CNCM n° I-4324
<400> 12
   Ala Leu Leu Gly Gly Gln Val Phe Asn Trp Asn Leu Pro Glu
   1               5                   10
<210> 13
   <211> 17
   <212> PRT
   <213> Artificial Sequence
<220>
   <223> CNCM n° I-4324
<400> 13
   Asx Leu Leu Glu Val Gly Asn Thr Gly Thr Phe Gly Ser Thr Gly Ser
   1               5                   10                  15
   Ala
<210> 14
   <211> 15
   <212> PRT
   <213> Artificial Sequence
<220>
   <223> CNCM n° I-4324
<400> 14
   Ala Leu Leu Gly Gly Gln Val Phe Asn Trp Asn Leu Pro Glu Ser
   1               5                   10                  15
<210> 15
   <211> 7
   <212> PRT
   <213> Artificial Sequence
<220>
   <223> CNCM n° I-4324
<400> 15
   Asx Glu Gln Leu Asn Phe Arg
   1               5
<210> 16
   <211> 9
   <212> PRT
   <213> Artificial Sequence
<220>
   <223> CNCM n° I-4324
<400> 16
   Leu Glu Leu Leu Asp Leu Glu Leu Glu
   1               5
<210> 17
   <211> 8
   <212> PRT
   <213> Artificial Sequence
<220>
   <223> CNCM n° I-4324
<400> 17
   Thr Gly Val Gly Ser Tyr Ala Arg
   1               5
<210> 18
   <211> 16
   <212> PRT
   <213> Artificial Sequence
<220>
   <223> CNCM n° I-4324
<400> 18
   Asx Pro Val Tyr Gly Asn Gln Val Gln Val Ser Phe Asp Leu Trp Arg
   1               5                   10                  15
<210> 19
   <211> 16
   <212> PRT
   <213> Artificial Sequence
<220>
   <223> CNCM n° I-4324
<400> 19
   Gly Gly Gly Gly Ser Asn Asp Pro Ala Leu Cys Leu Tyr Leu Ala Arg
   1               5                   10                  15
<210> 20
   <211> 9
   <212> PRT
   <213> Artificial Sequence
<220>
   <223> CNCM n° I-4324
<400> 20
   Ala Cys Pro Ser Ser Gly Val Phe Gln
   1               5
<210> 21
   <211> 6
   <212> PRT
   <213> Artificial Sequence
<220>
   <223> CNCM n° I-4324
<400> 21
   Leu Leu Ser Gly Trp Gly
   1               5
<210> 22
   <211> 8
   <212> PRT
   <213> Artificial Sequence
<220>
   <223> CNCM n° I-4324
<400> 22
   Asx Val Tyr Asp Asn Thr Leu Val
   1               5
<210> 23
   <211> 8
   <212> PRT
   <213> Artificial Sequence
<220>
   <223> CNCM n° I-4324
<400> 23
   Phe Gly Val Thr Gly Pro Pro Thr
   1 5
<210> 24
   <211> 8
   <212> PRT
   <213> Artificial Sequence
<220>
   <223> CNCM n° I-4324
<400> 24
   Asp Leu Leu Gly Asn Thr Leu Asp
   1 5
<210> 25
   <211> 9
   <212> PRT
   <213> Artificial Sequence
<220>
   <223> CNCM n° I-4324
<400> 25
   Asx Asp Thr Asp Leu Pro Asn Pro Arg
   1 5
<210> 26
   <211> 5
   <212> PRT
   <213> Artificial Sequence
<220>
   <223> CNCM n° I-4324
<400> 26
   Ala Thr Gly Ala Gly
   1 5
<210> 27
   <211> 15
   <212> PRT
   <213> Artificial Sequence
<220>
   <223> CNCM n° I-4324
<400> 27
   Asx Ser Cys Tyr Ala Asn Tyr Ser Glu Ser Ser Leu Leu Gly Lys
   1 5 10 15
<210> 28
   <211> 16
   <212> PRT
   <213> Artificial Sequence
<220>
   <223> CNCM n° I-4324
<400> 28
   Asx Asp Asp Pro Asn Asn Pro Gly Gln Thr Leu His Tyr Ala Trp Lys
   1 5 10 15
<210> 29
   <211> 10
   <212> PRT
   <213> Artificial Sequence
<220>
   <223> CNCM n° I-4324
<400> 29
   Asx Phe Trp Gly Leu Tyr Asn Leu Thr Asp
   1 5 10
<210> 30
   <211> 21
   <212> PRT
   <213> Artificial Sequence
<220>
   <223> CNCM n° I-4324
<400> 30
   Leu Leu Glu Val Gly Asn Thr Gly Thr Phe Gly Ser Thr Gly Ser Tyr
   1 5 10 15
   Leu Met Gln Ala Lys
   20
<210> 31
   <211> 14
   <212> PRT
   <213> Artificial Sequence
<220>
   <223> CNCM n° I-4324
<400> 31
   Asp Leu Ala Asn Pro Asp Asn Val Gly Thr Val Asp Asp Arg
   1 5 10
<210> 32
   <211> 16
   <212> PRT
   <213> Artificial Sequence
<220>
   <223> CNCM n° I-4324
<400> 32
   Gln Glu Met Ala Leu Leu Met Gln Glu Val Asp Trp Asn Leu Pro Glu
   1 5 10 15
<210> 33
   <211> 5
   <212> PRT
   <213> Artificial Sequence
<220>
   <223> CNCM n° I-4324
<400> 33
   Ala Asp Leu Tyr Arg
   1 5
<210> 34
   <211> 14
   <212> PRT
   <213> Artificial Sequence
<220>
   <223> CNCM n° I-4324
<400> 34
   Val Val Asp Asn Ser Thr Leu Pro Ala Ala Asp Leu Tyr Arg
   1 5 10
<210> 35
   <211> 5
   <212> PRT
   <213> Artificial Sequence
<220>
   <223> CNCM n° I-4324
<400> 35
   Ala Asp Leu Tyr Arg
   1 5
<210> 36
   <211> 17
   <212> PRT
   <213> Artificial Sequence
<220>
   <223> CNCM n° I-4324
<400> 36
   Tyr His Asp Thr Asn Asn Met Leu Thr His Ser Ala Asn Leu Asp Asp
   1               5                   10                  15
   Arg
<210> 37
   <211> 12
   <212> PRT
   <213> Artificial Sequence
<220>
   <223> CNCM n° I-4324
<400> 37
   Leu Tyr Glu Asn Gly Glu Leu Val Asp Glu Phe Leu
   1               5                   10
<210> 38
   <211> 23
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (6)..(6)
   <223> n is a, c, g, or t
<220>
   <221> misc_feature
   <222> (12)..(12)
   <223> n is a, c, g, or t
<220>
   <221> misc_feature
   <222> (15)..(15)
   <223> n is a, c, g, or t
<220>
   <221> misc_feature
   <222> (18)..(18)
   <223> n is a, c, g, or t
<400> 38
   gtnggnaaya cnggnacntt ygg 23
<210> 39
   <211> 20
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (12)..(12)
   <223> n is a, c, g, or t
<220>
   <221> misc_feature
   <222> (18)..(18)
   <223> n is a, c, g, or t
<400> 39
   ccnaaygayc cnaayytnaa 20
<210> 40
   <211> 20
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (9)..(9)
   <223> n is a, c, g, or t
<220>
   <221> misc_feature
   <222> (18)..(18)
   <223> n is a, c, g, or t
<400> 40
   gcnaayccng ayaaygtngg 20
<210> 41
   <211> 23
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (15)..(15)
   <223> n is a, c, g, or t
<220>
   <221> misc_feature
   <222> (21)..(21)
   <223> n is a, c, g, or t
<400> 41
   acnytnccng cngcngayyt nta 23
<210> 42
   <211> 21
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (6)..(6)
   <223> n is a, c, g, or t
<220>
   <221> misc_feature
   <222> (12)..(12)
   <223> n is a, c, g, or t
<400> 42
   ggnggncarg tnttyaaytg g 21
<210> 43
   <211> 24
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (21)..(21)
   <223> n is a, c, g, or t
<400> 43
   ggncaracny tncaytaygc ntgg 24
<210> 44
   <211> 23
   <212> DNA
   <213> Artificial Sequence
<220>
   <223> Amorce
<220>
   <221> misc_feature
   <222> (6)..(6)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (18)..(18)
   <223> n is a, c, g, or t
<220>
   <221> misc_feature
   <222> (21)..(21)
   <223> n is a, c, g, or t
<400> 44
   ccraangtnc cngtrttncc nac 23
<210> 45
   <211> 20
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (9)..(9)
   <223> n is a, c, g, or t
<220>
   <221> misc_feature
   <222> (18)..(18)
   <223> n is a, c, g, or t
<400> 45
   ttnarrttng grtcrttngg 20
<210> 46
   <211> 20
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (12)..(12)
   <223> n is a, c, g, or t
<220>
   <221> misc_feature
   <222> (18)..(18)
   <223> n is a, c, g, or t
<400> 46
   ccnacrttrt cnggrttngc 20
<210> 47
   <211> 23
   <212> DNA
   <213> Artificial Sequence
<220>
   <223> Amorce
<220>
   <221> misc_feature
   <222> (3)..(3)
   <223> n is a, c, g, or t
<220>
   <221> misc_feature
   <222> (9)..(9)
   <223> n is a, c, g, or t
<220>
   <221> misc_feature
   <222> (12)..(12)
   <223> n is a, c, g, or t
<220>
   <221> misc_feature
   <222> (15)..(15)
   <223> n is a, c, g, or t
<220>
   <221> misc_feature
   <222> (18)..(18)
   <223> n is a, c, g, or t
<220>
   <221> misc_feature
   <222> (21)..(21)
   <223> n is a, c, g, or t
<400> 47
   tanarrtcng cngcnggnar ngt 23
<210> 48
   <211> 21
   <212> DNA
   <213> Artificial Sequence
<220>
   <223> Amorce
<220>
   <221> misc_feature
   <222> (10)..(10)
   <223> n is a, c, g, or t
<220>
   <221> misc_feature
   <222> (16)..(16)
   <223> n is a, c, g, or t
<220>
   <221> misc_feature
   <222> (19)..(19)
   <223> n is a, c, g, or t
<400> 48
   ccarttraan acytgnccnc c 21
<210> 49
   <211> 24
   <212> DNA
   <213> Artificial Sequence
<220>
   <223> Amorce
<220>
   <221> misc_feature
   <222> (4)..(4)
   <223> n is a, c, g, or t
<220>
   <221> misc_feature
   <222> (13)..(13)
   <223> n is a, c, g, or t
<220>
   <221> misc_feature
   <222> (16)..(16)
   <223> n is a, c, g, or t
<220>
   <221> misc_feature
   <222> (22)..(22)
   <223> n is a, c, g, or t
<400> 49
   ccangcrtar tgnarngtyt gncc 24
<210> 50
   <211> 28
   <212> DNA
   <213> Artificial Sequence
<220>
   <223> Amorce
<400> 50
   ctaggttgta atgtgttagg tgcatccc 28
<210> 51
   <211> 26
   <212> DNA
   <213> Artificial Sequence
<220>
   <223> Amorce
<400> 51
   gtgaatcgcg cataacttcc cacacc 26
<210> 52
   <211> 24
   <212> DNA
   <213> Artificial Sequence
<220>
   <223> Amorce
<400> 52
   cccgtgtgct tacctttggc ctgc 24
<210> 53
   <211> 25
   <212> DNA
   <213> Artificial Sequence
<220>
   <223> Amorce
<400> 53
   gcagctggaa gaaccgaggt ctttc 25
<210> 54
   <211> 26
   <212> DNA
   <213> Artificial Sequence
<220>
   <223> Amorce
<400> 54
   ccggaaccag aacgaggaag agaatc 26
<210> 55
   <211> 26
   <212> DNA
   <213> Artificial Sequence
<220>
   <223> Amorce
<400> 55
   ggaggaagag cacaaatgag atgggc 26
<210> 56
   <211> 35
   <212> DNA
   <213> Artificial Sequence
<220>
   <223> Amorce
<400> 56
   cacgtaatct gggtaggttt ttatatcatg atacc 35
<210> 57
   <211> 26
   <212> DNA
   <213> Artificial Sequence
<220>
   <223> Amorce
<400> 57
   gcttctgtag gtgtgtatcc taaccc 26
<210> 58
   <211> 29
   <212> DNA
   <213> Artificial Sequence
<220>
   <223> Amorce
<400> 58
   gctggacgtg tgtcttcttt gtattacgc 29
<210> 59
   <211> 16
   <212> DNA
   <213> Artificial Sequence
<220>
   <223> Amorce
<220>
   <221> misc_feature
   <222> (5)..(5)
   <223> n is a, c, g, or t
<220>
   <221> misc_feature
   <222> (10)..(10)
   <223> n is a, c, g, or t
<400> 59
   tgwgnagwan casaga 16
<210> 60
   <211> 16
   <212> DNA
   <213> Artificial Sequence
<220>
   <223> Amorce
<220>
   <221> misc_feature
   <222> (5)..(5)
   <223> n is a, c, g, or t
<220>
   <221> misc_feature
   <222> (10)..(10)
   <223> n is a, c, g, or t
<400> 60
   agwgnagwan cawagg 16
<210> 61
   <211> 16
   <212> DNA
   <213> Artificial Sequence
<220>
   <223> Amorce
<220>
   <221> misc_feature
   <222> (5)..(5)
   <223> n is a, c, g, or t
<220>
   <221> misc_feature
   <222> (10)..(10)
   <223> n is a, c, g, or t
<220>
   <221> misc_feature
   <222> (13)..(13)
   <223> n is a, c, g, or t
<400> 61
   wgtgnagwan canaga 16
<210> 62
   <211> 15
   <212> DNA
   <213> Artificial Sequence
<220>
   <223> Amorce
<220>
   <221> misc_feature
   <222> (1)..(1)
   <223> n is a, c, g, or t
<400> 62
   ntcgastwts gwgtt 15
<210> 63
   <211> 16
   <212> DNA
   <213> Artificial Sequence
<220>
   <223> Amorce
<220>
   <221> misc_feature
   <222> (1)..(1)
   <223> n is a, c, g, or t
<220>
   <221> misc_feature
   <222> (11)..(11)
   <223> n is a, c, g, or t
<400> 63
   ngtcgaswga nawgaa 16
<210> 64
   <211> 8
   <212> PRT
   <213> Artificial Sequence
<220>
   <223> CNCM n° I-4324
<400> 64
   Val Gly Asn Thr Gly Thr Phe Gly
   1               5
<210> 65
   <211> 7
   <212> PRT
   <213> Artificial Séquence
<220>
   <223> CNCM n° I-4324
<400> 65
   Ala Asn Pro Asp Asn Val Gly
   1               5
<210> 66
   <211> 8
   <212> PRT
   <213> Artificial Sequence
<220>
   <223> CNCM n° I-4324
<400> 66
   Thr Leu Pro Ala Ala Asp Leu Tyr
   1               5
<210> 67
   <211> 7
   <212> PRT
   <213> Artificial Sequence
<220>
   <223> CNCM n° I-4324
<400> 67
   Gly Gly Gln Val Phe Asn Trp
   1               5
<210> 68
   <211> 8
   <212> PRT
   <213> Artificial Sequence
<220>
   <223> CNCM n° I-4324
<400> 68
   Gly Gln Thr Leu His Tyr Ala Trp
   1               5
<210> 69
   <211> 36
   <212> DNA
   <213> Artificial Sequence
<220>
   <223> CNCM I 4324
<400> 69
   ggggggagat ctgcgcctga tgaggataca aattct 36
<210> 70
   <211> 39
   <212> DNA
   <213> Artificial Sequence
<220>
   <223> CNCM I 4324
<400> 70
   cccccccaat tgttatcctg acgtacttgc gataatgct 39

## Description

### Domaine technique

La présente invention se rapporte notamment à des ulvanes lyases ayant une activité iduronique et glucuronique lyase extraite du microorganisme déposé le 17 juin 2010 sous le numéro I-4324 à la collection nationale de cultures de micro-organismes (CNCM) 25 rue du docteur Roux, 75724 Paris Cedex 15, France, de 46kD de séquence SEQ ID NO 4, à des séquences d'acides nucléiques codant pour ces ulvanes lyases, à des vecteurs comprenant ces séquences codantes, à un procédé de fabrication de ces ulvanes lyases, ainsi qu'à un procédé de dégradation d'ulvanes utilisant ces ulvanes lyases.

La présente invention trouve notamment des applications dans la valorisation des bio-ressources naturelles constituées par les organismes et les microorganismes comprenant des ulvanes, notamment les algues vertes. En particulier, elle trouve des applications en laboratoire, pour l'analyse de ces ulvanes, ainsi que dans l'agro-alimentaire, dans le domaine de la cosmétique et dans le domaine des médicaments et formulations pharmaceutiques ou les produits de dégradation des ulvanes sont valorisables.

Dans la description ci-dessous, les références entre crochets [ ] renvoient à la liste des références présentée à la fin du texte.

### Etat de la technique

Les algues vertes appartenant au genre Ulvales *(Ulva sp. et Enteromorpha sp.)* sont présentes partout sur le globe terrestre et sont très communément rencontrées sur les côtes. Ces algues sont fréquemment impliquées dans la prolifération algale favorisée par l'eutrophisation des eaux côtières donnant lieu aux « marées vertes ».

Jusqu'à présent cette biomasse indésirable avait une très faible valeur ajoutée et est utilisée essentiellement comme compost.

Les polysaccharides anioniques complexes présents dans la paroi des ulves, appelée ulvanes, possèdent des structures originales et représentent une source de biopolymères dont les fonctionnalités sont encore peu explorées.

Les ulvanes sont composés de différentes unités de répétition disaccharidiques construites avec des unités rhamnoses, acides glucuroniques, acides iduronique, xyloses et de sulfates. Les deux principaux motifs de répétition sont appelés acide aldobiuronique, ou acides ulvanobiuroniques, ou respectivement A (A_{3S}) et B (B_{3S}), dont les formules sont les suivantes :

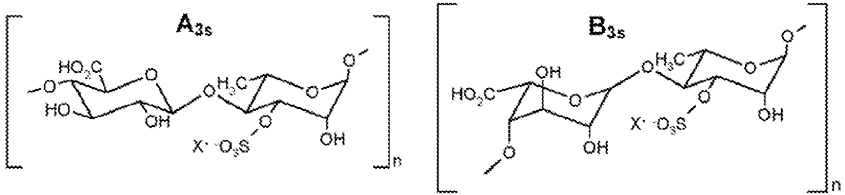

Le motif A (A_{3S}) est l'acide béta-D-1,4-glucuronique (1→4) alpha-L-1,4-rhamnose 3-sulfate. Le motif B (B_{3S}) est l'acide alpha-L-1,4-iduronique (1→4) alpha-L-1,4-rhamnose 3-sulfate.

Les acides uroniques sont parfois remplacés par des résidus xylose sulfatés en O-2.

Les ulvanes possèdent des propriétés physicochimiques uniques qui en font des candidats attractifs pour de nouvelles applications agroalimentaires, pharmaceutiques, et cosmétiques. Les ulvanes possèdent des structures très originales composées de sucres ou monosaccharides rares comme le rhamnose et l'acide iduronique. Le rhamnose est un composé important des antigènes de surface de nombreux microorganismes reconnus spécifiquement par les lectines de mammifères. Il est également utilisé pour la synthèse d'arômes. L'acide iduronique est utilisé pour la synthèse des glycosaminoglycanes, par exemple l'héparine.

En plus des monomères, les ulvanes et oligo-ulvanes présentent des propriétés biologiques intéressantes. En effet, des travaux ont montré, par exemple, que les oligo-ulvanes ont des activités antitumorales, antivirales, notamment antigrippales, et anticoagulantes. Une liste non-exhaustive d'applications potentielles des ulvanes a été proposée par M. Lahaye et A. Robic dans le document Structure and functional properties of ulvan, a polysaccharide from green seaweeds. Biomacromolecules 2007, 8, 1765-1774 [1].

Dans ce contexte, une meilleure compréhension de la structure des ulvanes et le développement de procédés permettant de fragmenter les ulvanes sous forme oligomérique ou monomérique revêt un très grand intérêt.

La demande international PCT WO 2009/016275 décrit une «substance enzymatique » purifiée à partir d'un microorganisme, à savoir la souche Ochrobactrum apte à venir couper la liaison osidique par β-élimination. En particulier, cette demande décrit uniquement une « solution » ou « milieu » comprenant une activité enzymatique. Il s'agit notamment de l'obtention de la substance enzymatique à partir *d'Ochrobactrum tritici* qui provient du sol.

Le document Lahaye M et al: "Fine chemical structure analysis of oligosaccharides produced by an ulvane-lyase degradation of the water-soluble cell-wall polysaccharides from Ulva sp." Carbohydrate Research, Pergamon, GB, Vol.304, n°3-4, 28 novembre 1997, p.325-333; décrit une bactérie possédant une activité enzymatique dégradant les ulvanes constituée par un mélange de protéines. Il décrit également l'isolement d'une bactérie marine à partir de boues et un procédé de dégradation d'ulvan comprenant l'incubation des bactéries dans un milieu de culture et l'ajout de solution d'ulvan dans le milieu de culture et la mesure des constantes catalytiques.

Actuellement, faute de moyens permettant de les connaître mieux et de les dégrader efficacement, les algues, notamment les algues vertes, sont essentiellement compostées et aucune valorisation industrielle n'en est faite. Ceci est d'autant plus déplorable que la source est abondante et parfois gênante en termes de pollution de nos côtes maritimes. Leur élimination est actuellement réalisée par voie de compost.

Il existe donc un réel besoin de trouver de nouveaux moyens de dégradation des ulvanes afin de pouvoir valoriser cette bio-ressource, issue notamment des algues vertes, en produisant des fragments oligo-ulvanes à façon dans la perspective d'applications cosmétiques, agro-alimentaires et médicales.

### Exposé de l'invention

La présente invention a précisément pour but de répondre à ce besoin en fournissant des ulvanes lyases qui dégradent très efficacement les ulvanes par dépolymérisation. L'étude des modalités de reconnaissance des enzymes de la présente invention réalisée par les inventeurs démontre leur activité glucuronique lyase.

Les inventeurs ont également démontré l'activité iduronique et glucuronique lyase.

Les inventeurs fournissent en particulier des ulvanes lyase extraites du microorganisme déposé sous le numéro I-4324 à la collection nationale de cultures de micro-organismes (CNCM) 25 rue du docteur Roux, 75724 Paris Cedex 15, France. Ce microorganisme est appelé également dans la présente et dans les documents de dépôt à la CNCM « 01-PN-2010 ».

En d'autres termes, Les inventeurs fournissent en particulier une ulvane lyase extraites du microorganisme d'origine marine déposé sous le numéro I-4324 à la collection nationale de cultures de micro-organismes (CNCM) 25 rue du docteur Roux, 75724 Paris Cedex 15, France, de 46kD de séquences SEQ ID NO 4.

Des ulvanes lyase, appelées aussi protéines ulvanolytiques, sont également décrites et peuvent être par exemple de 30 ou 46kD et comprendre dans leur séquence peptidique les quatre séquences suivantes :
PNDPNLK (SEQ IDn°9),
LLEVGNTGTFGSTGS (SEQ IDn°10),
DLANPDNV (SEQ IDn° 11), et
WNLPE (SEQ IDn°12).

Les inventeurs ont en effet isolé notamment des ulvanes lyases de 30 kD ou 46 kD présentant ces séquences en commun dans leur séquence respective. Bien que ces protéines possèdent des fragments peptidiques communs entre elles, leurs séquences n'ont jamais été décrites dans l'art antérieur. Ces séquences de 30 ou 46 kD sont actives pour dégrader les ulvanes par dépolymérisation selon la présente invention. Elles ont été extraites comme indiqué dans les exemples ci-dessous.

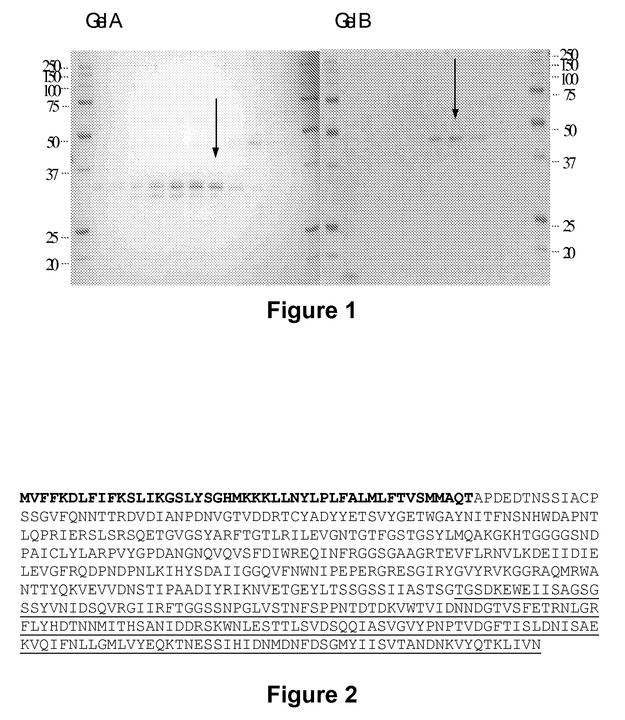

L'une de ces séquences est la séquence SEQ ID n°1 de 30 kD. La présente décrit donc également à cette séquence. Cette séquence est la partie catalytique d'une ulvane lyase isolée par les inventeurs et de 46 kD (SEQ IDn°4). Cette ulvane lyase est représentée sur la figure 2 annexée.

La séquence ID n°1 peut donc comprendre à son extrémité C-terminale, la séquence SEQ IDn°2 du listage de séquences annexé.

Selon l'invention, ces ulvanes lyases, quelle que soit leur séquence, peuvent comprendre en outre, à leur extrémité N-terminale, une séquence signal ou séquence d'adressage. Cette séquence signal peut être une des séquences signal connues de l'homme du métier pour que la protéine, lorsqu'elle est synthétisée dans une cellule hôte, soit dirigée vers un organite ou une zone particulière de la cellule hôte. Il peut s'agir par exemple d'une séquence signal trouvée dans les sites spécialises dans la prédiction de peptides signaux par exemple http://www.cbs.dtu.dk/services/SignaIP/ [2] ou encore http://bmbpcu36.leeds.ac.uk/prot_analysis/Signal.htmL [3]. Il peut s'agir par exemple la séquence SEQ IDn°3 du listage de séquences annexé. Cette séquence signal peut être clivée après synthèse de la protéine ou non. Les procédés de clivage connus de l'homme du métier peuvent être utilisés, par exemple ceux impliquant des protéases spécifiques d'un site de clivage. On choisi alors une séquence signal présentant un tel site. La présente décrit des acides nucléiques codant pour les ulvanes lyases, notamment pour la protéine SEQ IDn°1. Il peut s'agir par exemple d'un acide nucléique comprenant ou constitué de la séquence SEQ IDn°5 du listage de séquences annexé.
La présente décrit également à un acide nucléique codant pour la protéine de séquence SEQ IDn°2 du listage de séquences annexé. Il peut s'agir par exemple de la séquence SEQ IDn°6 du listage de séquences annexé.

La présente décrit également à un acide nucléique codant pour la protéine de séquence SEQ IDn°3 du listage de séquences annexé. Il peut s'agir par exemple de la séquence SEQ IDn°7 du listage de séquences annexé.

La présente invention se rapporte également à un acide nucléique codant pour la protéine de séquence SEQ IDn°4 du listage de séquences annexé. Il peut s'agir par exemple de la séquence SEQ IDn°8 du listage de séquences annexé.

Ces séquences d'acides nucléiques ou gènes de la présente invention sont les premiers représentants d'une nouvelle famille de gènes codant pour des polysaccharides-lyases et représentent également les premiers gènes d'enzymes conformes à la présente invention de dégradation de l'ulvane.

La présente invention se rapporte également à un vecteur comprenant un acide nucléique codant pour une des ulvanes lyases de la présente invention :, un acide nucléique de séquences SEQ ID ou 8. Le vecteur peut être un des vecteurs connus de l'homme du métier pour fabriquer des protéines par recombinaison génétique. Il est choisi en général notamment en fonction de l'hôte cellulaire choisi. Le vecteur peut être par exemple choisi parmi les vecteurs listés dans le catalogue http://www.promega.com/vectors/mammalian_express_vectors.htm [4] ou http://www.qiagen.com/overview/qiagenes.aspx?gaw=PROTQIAgenes080 7&gkw=mammalian+expression [5], ou encore http://www.scbt.com/chap_exp_vectors.php?type=pCruzTM%20Expressio n%20Vectors [6]. Il peut s'agir par exemple du vecteur d'expression décrit dans le document WO 83/004261 [7].

Les acides nucléiques de la présente invention ou les vecteurs de la présente invention sont utilisables notamment pour la fabrication par recombinaison génétique des ulvanes lyases de la présente invention. Aussi, la présente invention se rapporte également à une cellule hôte comprenant une séquence d'acide nucléique selon l'invention ou un vecteur selon l'invention.

La cellule hôte ou hôte cellulaire peut être tout hôte approprié pour la fabrication des ulvanes lyase de la présente invention à partir des acides nucléiques ou des vecteurs de l'invention. Il peut s'agir par exemple *d'E. coli,* de *Pischia pastoris,* de *Saccharomyces cerevisiae,* de cellules d'insectes, par exemple un système cellules d'insectes-baculovirus (par exemple cellules d'insecte SF9 utilisant un système d'expression de baculovirus), de mammifères.

Aussi, la présente invention se rapporte également à un procédé de fabrication d'une ulvane lyase selon l'invention par recombinaison génétique utilisant un acide nucléique ou un vecteur selon l'invention. Les procédés de recombinaison génétique connus de l'homme du métier sont utilisables, L'origine marine ou terrestre n'influence pas la possibilité de recombinaison et d'expression hétérologue.

Les inventeurs de la présente sont en outre les tout premiers à avoir isolé un microorganisme, nommé par eux 01-PN-2010, qui produit les ulvanes lyase de la présente invention. Il s'agit d'une bactérie marine. Cette bactérie marine se trouve par exemple dans les fèces *d'Aplysia punctata (Mullusca, Gastropoda).* Ils ont déposé cette bactérie conformément au Traité de Budapest sur la reconnaissance internationale du dépôt des micro-organismes aux fins de la procédure en matière de brevets à la collection nationale de cultures de micro-organismes (CNCM) 25 rue du docteur Roux, 75724 Paris Cedex 15, France. Le numéro de dépôt de cette souche à la CNCM est I-4324.

Les inventeurs ont purifié à partir de ce microorganisme les deux ulvanes lyases de 30 kD et 46 kD décrites dans la présente.

Aussi, la présente invention se rapporte également au microorganisme déposé sous le numéro I-4324 à la collection nationale de cultures de micro-organismes (CNCM) 25 rue du docteur Roux, 75724 Paris Cedex 15, France.

Ce microorganisme peut donc être utilisé notamment pour la fabrication d'ulvanes lyase. La présente décrit donc également à un procédé de fabrication d'ulvanes lyase comprenant la mise en culture du microorganisme déposé sous le numéro I-4324 à la CNCM en France.

Cette mise en culture se fait de préférence dans un milieu de culture permettant la croissance de ce microorganisme marin. Il peut s'agir par exemple milieu de culture liquide ZoBell, comme décrit dans le document ZoBell, CE 1941 Studies on marine bacteria. I. The cultural requirements of heterotrophic aerobes, J Mar Res 4, 41-75 [8]. Des conditions de culture utilisables pour la mise en oeuvre de la présente invention sont également décrites dans ce document. Le pH de culture est de préférence compris entre 7 et 9, de préférence pH 8. La température de culture est de préférence comprise entre 15 et 30°C, de préférence 25°C. La culture est de préférence réalisée avec une concentration NaCl de 20 à 30 g.L⁻¹, de préférence de 25 g._{L}-¹.

Ce procédé de fabrication des ulvanes lyases en utilisant le microorganisme de l'invention ou tout autre cellule hôte transformée pour une fabrication par recombinaison génétique conformément à la présente invention, peut comprendre en outre une étape de récupération des ulvanes lyases. Cette étape de récupération ou d'isolement peut être réalisée par tout moyen connu de l'homme du métier. Il peut s'agir par exemple d'une technique choisie parmi une électrophorèse, un tamisage moléculaire, une ultracentrifugation, une précipitation différentielle, par exemple au sulfate d'ammonium, par ultrafiltration, une filtration sur membrane ou sur gel, un échange d'ions, une élution sur hydroxyapatite, une séparation par interactions hydrophobes, ou tout autre moyen connu. Un exemple de procédé d'isolement de ces ulvanes lyases utilisable pour la mise en oeuvre de la présente invention est décrit ci-dessous.

Le microorganisme précité ou toute autre cellule hôte transformée pour une fabrication par recombinaison génétique conformément à la présente invention peut également être utilisé directement pour dégrader des ulvanes, dans leur milieu naturel ou en culture. Lorsqu'il s'agit d'une culture, il peut s'agir d'un système discontinu ou continu. On peut utiliser par exemple un réacteur de culture contenant un milieu de culture approprié au développement du microorganisme.

La présente invention se rapporte donc également à un procédé de dégradation d'ulvanes comprenant une étape de mise en contact des ulvanes avec une ulvane lyase selon l'invention ou avec une cellule hôte selon l'invention ou avec le microorganisme déposé sous le numéro I-4324 à la CNCM en France, dans les conditions permettant la dégradation des ulvanes par digestion enzymatique par ladite ulvane lyase ou ladite cellule hôte transformée ou ledit microorganisme déposé sous le numéro I-4324 à la CNCM en France.

Les conditions permettant la dégradation des ulvanes, lorsqu'on utilise une cellule hôte ou microorganisme, sont celles exposées ci-dessus, respectivement pour la cellule hôte ou pour le microorganisme.

Pour la digestion enzymatique, la détermination des constantes de Michaelis Menten (Km et Vmax) permet aisément à l'homme du métier de trouver les conditions optimales de concentration de l'ulvane lyase utilisée et de concentration des ulvanes pour la dégradation des ulvanes dans le milieu où elles se trouvent ou dans le milieu dans lequel elles ont été placées. Le pH peut être également compris de préférence entre 7 et 8, de préférence entre 9 et 9,5. Il s'agit en effet de la gamme de pH optimale. La température (optimale) est de préférence comprise entre 30°C et 40°C. La force La force ionique supérieure à 300 mM NaCl pour la protéine de 46 kD et sans sel avec la protéine de 30 kD, dans un autre mode, la force ionique optimale est de 300 mM NaCl pour la protéine de 46 kD et 100 mM NaCl pour la protéine de 30 kD.

L'invention permet avantageusement de mobiliser la très grande ressource d'algues actuellement inexploitée, notamment d'algues vertes. L'invention permet en outre de favoriser la biodégradation des algues, notamment des algues vertes, de produire des molécules originales, qui sont des fragments d'ulvanes ou oligo-ulvanes, par exemple des oligosaccharides, par exemple aussi des hydrocolloïdes, et d'offrir une source nouvelle de monosaccharides rares pour des applications cosmétiques, agro-alimentaires et médicaments ou formulations pharmaceutiques et parapharmaceutiques.

Les produits de dégradation des ulvanes donnent accès à de nouveaux produits qui peuvent être des actifs alimentaires, cosmétiques, pharmaceutiques et parapharmaceutiques utilisables dans les domaines agro-alimentaires, cosmétiques, pharmaceutiques et parapharmaceutiques. Ces nouveaux produits peuvent également être des produits non actifs mais présentant une neutralité et/ou une stabilité qui est très intéressante pour une utilisation dans chacun de ces domaines.

L'utilisation des ulvanes lyases de la présente invention donne en outre accès à des monosaccharides rares utilisables comme synthons en glycochimie. La dégradation de l'ulvane avec les ulvanes lyase combinée à d'autres enzymes peut donner accès à l'acide iduronique (sucre rare) utilisé pour la synthèse des glycoaminoglycans de synthèse.

La présente invention ouvre également de nouvelles perspectives d'utilisation de ces algues pour des applications en bioénergie et en chimie. La production de fragments oligosaccharides peut donner de molécules de base pour la fabrication d'autres molécules. La dépolymérisation de l'ulvane devrait faciliter la fermentation par des microorganismes conduisant à la production de méthane par exemple.

D'autres caractéristiques et avantages apparaîtront encore à l'homme du métier à la lecture des exemples ci-dessous, donnés à titre illustratif et non limitatif, en référence aux figures annexées.

### Brève description des figures

- La figure 1 est un gel d'électrophorèse des protéines ulvanolytiques de 30kD (Gel A) et de 46 kD (Gel B). Les flèches indiquent les bandes qui ont été excisées puis utilisées pour le séquençage des peptides par spectrométrie de masse.
- La figure 2 représente la séquence protéique d'ulvane lyase de 46Dka (SEQ IDn°4) de la présente invention avec en gras le peptide ou séquence signal (SEQ IDn°3). En texte normal, le module catalytique, déterminé par spectrométrie de masse, SEQ IDn°1. La partie de la séquence protéique non catalytique (SEQ IDn°2) de la protéine 46kD est soulignée.

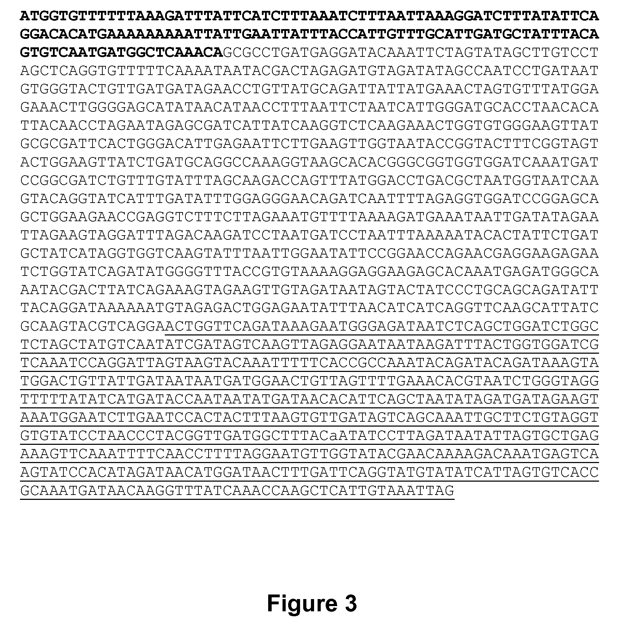

- La figure 3 représente la séquence du gène codant la protéine ulvane lyase de 46Dka. En gras le gène codant pour le peptide ou séquence signal (SEQ IDn°7). En texte normal, le gène SEQ IDn°5 codant pour le module catalytique de SEQ IDn°1, déterminé par spectrométrie de masse. En souligné, la séquence SEQ IDn°6 codant pour la partie non catalytique de la protéine de 46kD (SEQ IDn°2) présente dans le peptide 46kD.

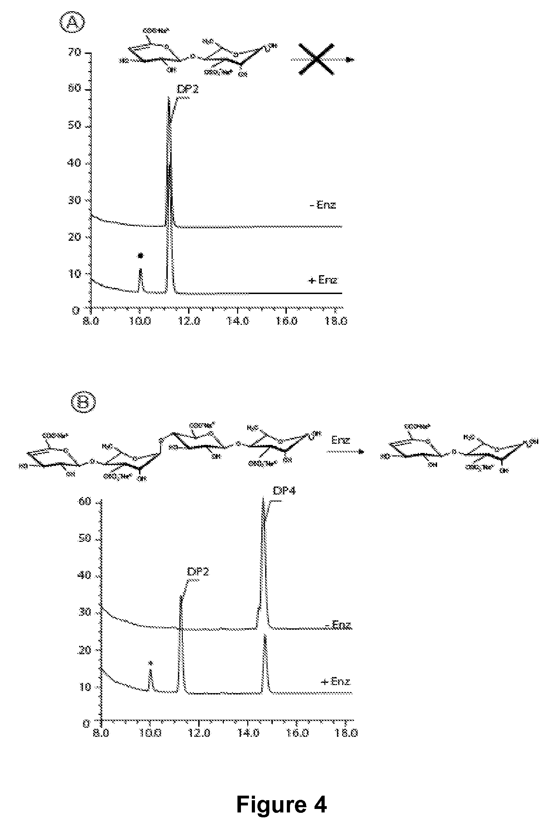

- La figure 4 représente les résultats obtenus d'expériences de chromatographie échangeuse d'ions conduites avant ou après incubation d'un disaccharide (graphique A) ou d'un tétrasaccharide (graphique B) avec l'ulvane lyase de la présente invention. Sur ces graphiques, l'axe des abscisses représente le temps d'élution en minute (min). et l'axe des ordonnées représente la conductimétrie en nano-Coulomb (nC).

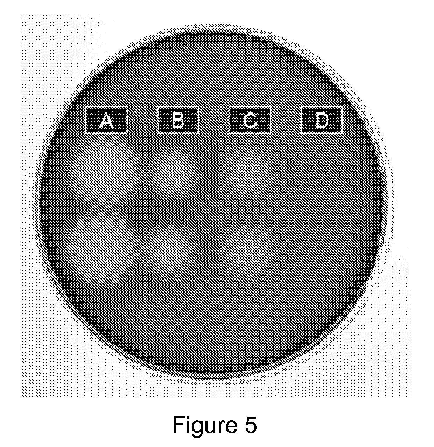

- La figure 5 est un photographie représentant des boites de Pétri avec des ulvanes marquées par une solution aqueuse de rouge de ruthénium à 0,05% montrant la dégradation enzymatique des ulvanes par l'ulvane lyase purifiée de 30 kD **(A),** 46 kD **(B)** et le module catalytique recombinant **(C).** Un extrait comprenant des bactéries *E. coli* dépourvue de plasmide d'expression a été également déposé sur l'agar comme contrôle **(D).**

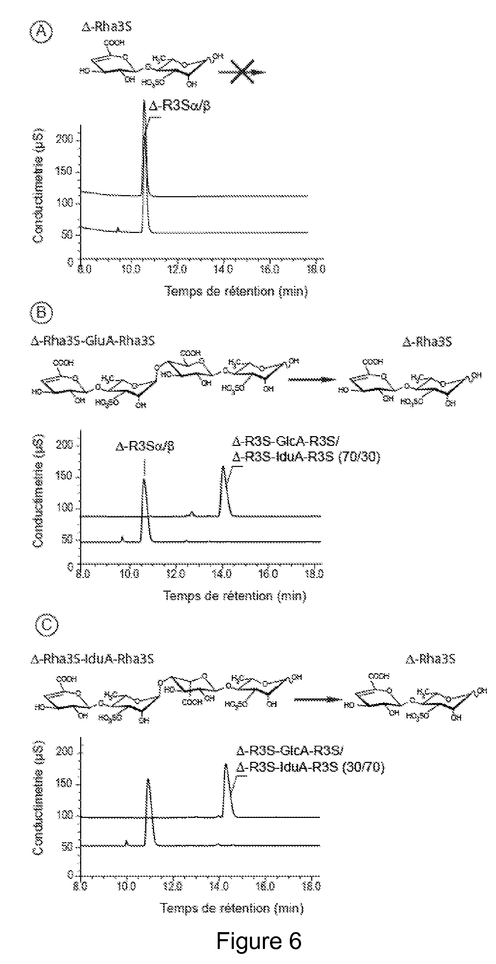

- La figure 6 représente les résultats obtenus d'expériences de chromatographie haute performance échangeuse d'anions d'oligosaccharides purifiés. **(A)** Δ-R3S n'a pas été dégradé par l'ulvanlyase. (B) Le tétrasaccharide Δ-R3S-Glc-R3S a été converti uniquement en Δ-R3S après incubation avec l'ulvan-lyase. **(C)** Le tétrasaccharide Δ-R3S-Idu-R3S a été converti uniquement en Δ-R3S après incubation avec l'ulvan-lyase comme observe aussi en **(B).** Sur ces graphiques, l'axe des abscisses représente le temps d'élution en minute (min). et l'axe des ordonnées représente la conductométrie en micro Siemens (µS).

### EXEMPLES

### Exemple 1: identification du microorganisme de la présente invention

Plusieurs individus *d'Aplysia punctata* (Mollusca, Gastropoda) ont été nourris avec des algues vertes appartenant au genre Ulva (Ulva sp.), en particulier *Ulva armoricana.* Les fèces des mollusques ont été collectés puis congelées pour stockage à -80°C.

Une fraction des fèces a été récoltée et a servi d'inoculum dans un milieu de culture POPSO. En particulier, cette fraction de fèces a été incubée sous agitation, rotation à 200 rpm, dans un incubateur qui contrôle température et rotation à 20°C pendant 72 heures dans 5 mL de milieu de culture POPSO (acide piperazine-N,N'-bis[2-hydroxypropane-3-sulfonique]) dont la composition est la suivante :
- 25 mM du tampon POPSO pH 7,0,
- 50 mM NaCl,
- 0,4 g d'acide casamino,
- 3 g d'ulvane
- 1 L d'Eau de mer.

L'ulvane utilisée ici et dans les exemples ci-dessous a été préparée par le procédé décrit dans le document Lahaye M. et al., Procédé d'extraction des ulvanes, (1996) Hydrobiologia, 326/327, 473 [9].

Une autre fraction des fèces a été incubée sous agitation pendant 72h dans 5mL de milieu de culture ZoBell 2216E (ZoBell, 1941) (voir ZoBell et al [8]). Le milieu ZoBell utilisé comprend les éléments suivants (pour 1 L de milieu ZoBell) : 5g de bactotryptone, 1g d'extrait de levure, 200 mL d'eau distillée et 800 mL d'eau de mer.

Chacune des cultures liquides ont ensuite été étalées sur des boîtes d'agar contenant du milieu de culture Zobell enrichi en ulvane ou « boites d'agar-Zobell-ulvane ». Ces milieux ZoBell solidifiés comprenaient outre la composition ci-dessus, 1,5% en poids d'agar (15g) et 0,4% en poids d'ulvane. Les boîtes ZoBell-agars ont été maintenues à 20°C pendant une semaine.

Après une semaine de culture à 37°C, sont apparues des colonies possédant des caractéristiques phénotypiques très différentes (couleur, la taille, forme, etc.). Ces colonies ont été isolées et repiquées plusieurs fois sur boites d'agar-Zobell-ulvane, avec à chaque fois une culture pendant une durée permettant de voir les colonies à une température de 20°C. Une vingtaine de souches ont été isolées.

Toutes les souches isolées ont été cultivées pendant 24 heures à 20°C en milieu de culture liquide Zobell enrichi avec 0,4% en poids d'ulvane. Après centrifugation à 1000 x g, les culots bactériens ont été lysés à l'aide d'une presse de French ou par un tampon de lyse chimique puis centrifugés à 1000 x g.

Les surnageants de cultures bactériennes et les surnageants des lysats bactériens ont été examinés séparément après avoir été incubés en présence d'ulvane. Pour cela, 50 µL de surnageant de culture bactérienne ou 5 µL de lysat bactérien ont été ajoutés à chaque fois à 1 mL de milieu réactionnel comprenant en % en poids : 1% d'ulvane, 200 mM NaCl dans 20 mM Tris-HCl, pH 7,7.

La formation de double-liaison produite par les activités des ulvanes lyases éventuellement présentes dans les extraits a été suivie par spectrophotométrie à 235 nm et la dépolymérisation du polysaccharide a été observée par électrophorèse (C-PAGE) et par chromatographie de filtration de gel.

Quatre des vingt souches isolées présentaient une très forte activité ulvanolytique dans les lysats bactériens et les surnageant de culture.

La souche la plus active a été référencée sous le nom de 01-PN-2010 et a ensuite été utilisée pour la purification des ulvanes lyases (exemples 2 et 3). Cette souche a été déposée à la collection nationale de cultures de micro-organismes (CNCM) 25 rue du docteur Roux, 75724 Paris Cedex 15, France, sous le numéro I-4324.

### Exemple 2 : Recherche des conditions optimales de culture de la souche 01-PN-2010 de la présente invention et caractérisation de la souche

Différentes cultures de la souche 01-PN-2010 ont été réalisées dans un milieu ZoBell 2216E, comme dans l'exemple 1, à des températures de 4, 10, 20, 30, 37 et 42°C pendant 24 h. La croissance de 01-PN-2010 a été observée par mesure de la densité optique à 600nm, en utilisant un spectrophotomètre Shimadzu (marque de commerce). Une croissance est observée d'environ 4°C à 35°C, l'optimum de croissance est entre 20 et 30, et plutôt autour de 25°C.

Le pH optimum de culture a été déterminé à 20°C sur milieu ZoBell en ajoutant différents tampons :
- 20 mM de tampon MES pour les pH 5,5 ; 6 et 6,5 ;
- 20 mM de tampon MOPS pour pH 7 ;
- 20 mM de tampon HEPES pour pH 7,5
- 20 mM de tampon TRIS-HCl pour pH 8 et 8,5 ;
- 20 mM tampon CHES pour pH 9 ; 9,5 et 10.

Une croissance de 01-PN-2010 a été observée entre pH 6,5 et 9 après 3 jours d'incubation, avec un optimum de croissance à pH 7,5 à 8.

L'effet de NaCl sur la croissance a également été testé sur milieu ZoBell, à 20°C et pH 8, avec différentes concentrations de NaCl : 0 ; 0,25 ; 0,5 ; 1,0 ; 2,0 ; 3,0 ; 4,0 ; 5,0 ; 6,0 ; 8,0 ; 10, 25, 30, 40, 50, 55, 60, 65, 70 et 80 g.l⁻¹. Après 2 jours d'incubation, une croissance a été observée dans les milieux ZoBell comprenant de 2,0 à 65 g.l⁻¹ de NaCl, avec un optimum se situant autour de 25 g.l⁻¹ (2,5% poids/volume).

Dans les conditions optimales identifiées suivantes :
- température de culture de 15 à 30°C, de préférence 15 à 25°C, de préférence 25°C ;
- pH entre 7 et 10, entre 7 et 9, de préférence pH 8 ;
- 20 à 30 g.L⁻¹ de NaCl, de préférence 25 g.L⁻¹ de NaCl, le temps de doublement de la souche est de 3 heures.

Les colonies sur un milieu ZoBell 2216E Agar en boîte de pétrie sont circulaires, luisantes, oranges, avec un diamètre de 2 à 3 mm après 5 jours d'incubation à 20°C. La culture de la souche sur milieu ZoBell-ulvane en boîte de pétrie pendant 24 heures à 20°C provoque la formation d'un trou qui résulte de l'hydrolyse de l'ulvane par la souche.

### Exemple 3 : purification d'une ulvane lyase selon l'invention de 30kD

La purification a été réalisée à partir d'une culture de 1 L de milieu de Zobell (voir ci-dessus pour la composition) avec 0,4% en poids d'ulvane inoculé avec 50 mL d'une culture fraîche de 01-PN-2010 possédant une densité optique à 600 nm.

La culture a été conduite en Erlenmeyer et maintenue à 20°C pendant 96 heures sous une agitation de 230 rpm (appareil, voir ci-dessus). Tout au long de la croissance bactérienne l'activité ulvanolytique a été suivie par spectrométrie à 235 nm.

Les bactéries ont été éliminées du milieu de culture par centrifugation à 7500 x g, 30 min à 10°C. Le surnageant a été concentré jusqu'à 120 mL par ultrafiltration à flux tangentielle sur un filtre de 10 kD (Prep/Scale (marque de commerce)-TFF, Milipore).

Une fraction des protéines a été précipitée par ajout de 1M (NH4)₂SO₄ par petite quantité sous agitation douce de la solution maintenue dans la glace.

Le précipité a été éliminé après centrifugation à 20 000 x g à 10°C et le surnageant possédant l'activité ulvanolytique a été utilisé pour la suite de la purification.

La totalité du surnageant a été injecté sur une colonne HiTrap phenyl-sepharose high sub (1 mL; GE Healthcare) équilibrée avec tampon A [20mM Tris-HCl pH 7,5, 1M (NH₄)₂SO₄] à un débit de 1 mL min⁻¹ à la température ambiante, ici 22°C. Le gel a été lavé avec 2 volumes de colonne de tampon A.

L'élution des protéines a été réalisée en appliquant un gradient linéaire décroissant de 1 M à 0 M (NH₄)₂SO₄ [tampon A sans (NH₄)₂SO₄ respectivement] sur 20 volumes de colonne.

Les fractions actives (15 mL) ont été réunies puis dessalées sur une colonne de dessalage HiPrep (2,6 x 30cm ; GE Healthcare) équilibré dans tampon B [20 mM Tris-HCL, pH 8,0]. Le débit de chargement et d'élution était de 3 mL min⁻¹.

L'échantillon dessalé a été chargé sur une colonne HiTrap Q FF (1 mL ; GE Healthcare) équilibrée avec tampon B. Le gel a été lavé avec de 2 volumes de colonne de tampon B avant élution avec un gradient linéaire de NaCl dans tampon B de 0 M à 1 M sur 20 volumes de colonne.

Les fractions actives ont été réunies et chargées sur une colonne HiTrap héparine HP (1 mL ; GE Healthcare) équilibré avec tampon C [tampon de phosphate de 10 mM, pH 7.0] à un débit de 1 mL min⁻¹.

Le gel a été lavé avec 2 volumes de colonne de tampon C avant élution avec un gradient linéaire de NaCl dans tampon C de 0 M à 1 M sur 20 volumes de colonne.

Les fractions actives (5 mL) ont été rassemblées et chargées sur une colonne du Superdex 75 HiPrep (1,6 x 60 cm ; GE Healthcare) équilibrée avec le tampon B avec 100 mM NaCl.

Les protéines ont été éluées par un gradient isocratique avec le même tampon à 1 mL min⁻¹.

Les inventeurs ont ainsi isolé une protéine de 30 kD. Cette protéine est une ulvane lyase active. Ils ont observé une petite contamination par deux autres protéines moins abondantes et de poids moléculaires très proches.

La protéine majoritaire de 30kD a été digérée par la trypsine, puis les peptides obtenus ont été analysés par spectrométrie de masse sur la plate-forme RIO "Biopolymères" localisée à l'INRA de Nantes. Les résultats d'analyse et de séquençage sont présentés dans le tableau 1 de l'exemple 5 ci-dessous.

### Exemple 4 : purification d'une autre ulvane lyase selon l'invention

La purification de cette autre ulvane lyase a été réalisée à partir d'une culture de 01-PN-2010 en fermenteur de 5 L dans le milieu de ZoBell décrit ci-dessus avec 0,4% en poids d'ulvane, à 25°C et avec un pH maintenu à pH 7,8 pendant 48 heures.

Les bactéries ont été éliminées par centrifugation à 8000 g, 1 h à 10°C. Le surnageant a été concentré jusqu'à 220 mL par ultrafiltration tangentielle sur le système milipore (Prep/Scale (marque de commerce)-TFF) avec un filtre de 10 kD. Comme précédemment, les protéines ont précipitées par ajout de 1 M (NH₄)₂SO₄.

Le culot a été éliminé après centrifugation à 20 000 g, 30 min à 10°C.

30 mL résine phénylsepharose 6 high sub équilibrée avec tampon B avec 1 M (NH₄)₂SO₄ ont été ajoutés au surnageant et maintenue pendant 30 min sous agitation douce. La résine a été séparée du milieu par filtration sur verre fritté. La résine a ensuite été lavée avec deux volumes de 75 mL de tampon B avec 1 M (NH₄)₂SO₄ et l'élution a été effectuée avec tampon B dans des aliquotes de 25 mL sur le verre fritté.

La suite de la purification a été conduite d'une manière similaire que pour la protéine de 30 kD dans l'exemple précédent. Les fractions actives (100 mL) ont été chargées sur une colonne HiTrap phénylsepharose high sub (1 mL ; GE Healthcare) équilibré dans tampon B avec 1 M (NH₄)₂SO₄ à un débit de 1 mL min⁻¹ à température ambiante, ici 22 ou 20°C.

Les fractions actives (30 mL) ont été mélangées et dessalées sur une colonne de dessalage HiPrep (2,6x30cm ; GE Healthcare) équilibrés dans tampon D [20mM Bis-Tris pH 6,0].

L'échantillon dessalé a été chargé sur une colonne HiTrap DEAE (1 mL ; GE Healthcare) équilibré avec tampon D à 1 mL min⁻¹. Le gel a été lavé avec 5 volumes de colonne de tampon D avant l'élution avec un gradient linéaire de NaCl de jusqu'à 1 M dans du tampon D.

Les fractions actives ont été mélangées (7 mL) et diluées à 50 mL dans tampon B avant chargement sur une colonne HiTrap Q FF (1 mL ; GE Healthcare) équilibré avec tampon B. Le gel a été lavé avec 5 volumes de colonne de tampon B avant l'élution avec un gradient linéaire de NaCl de jusqu'à 1 M dans tampon B.

Les fractions actives (5 mL) ont été mises et injectées en commun sur une colonne Superdex 75 HiPrep (1,6 x 60 cm ; GE Healthcare) équilibré avec tampon B avec 100mM NaCl.

Les protéines ont été éluées par un gradient isocratique avec le même tampon à 1 mL min⁻¹.

Les fractions actives (15 mL) ont été mélangées et dialysées sur la nuit contre tampon C.

L'échantillon dessalé a été chargé sur colonne HiTrap héparine HP préemballé (1 mL ; GE Healthcare) équilibré avec Tampon C [10mM tampon de phosphate pH 7,0] à un débit de 0,5 mL min⁻¹. Le gel a été lavé avec 2 volumes de colonne de Tampon C avant l'élution avec un gradient linéaire de NaCl dans tampon C de 0 M à 1 M sur 20 volumes de colonne.

A chaque étape de la purification, les fractions actives ont été analysées par SDS-PAGE suivant la technique décrite dans le document LaemmLi UK and Favre M 1973 Links maturation of the head of bacteriophage T4.I. DNA packaging events. J Mol Biol 80:575-599 [10].

La quantification de protéines a été exécutée selon la technique décrite dans le document Bradford MM 1976; A rapid and sensitive method for the quantitation of microgram quantities of protein using the principle of protein-dye binding. Anal Biochem 72: 248-254 [11] utilisant le réactif Biorad [Biorad protein assay (marque de commerce)] avec de l'albumine de sérum de boeuf en tant que standard.

L'activité de ulvane lyase extraite a été analysée par une méthode spectrophotométrique : 25-50 µL d'extrait est ajouté à 1 mL milieu réactionel [20 mM Tris-HCL pH 7 ou 8,5, 200 mM NaCl et 1% en poids d'ulvane]. L'augmentation de l'absorbance à 235 nanomètre a été suivie pendant 5 mn.

Pour l'identification des fractions actives, les inventeurs ont utilisé une méthode de détection sur boîte de pétrie suivant la méthode décrite dans le document Gacesa P and Wusteman FS 1990 Plate assay for simultaneous detection of alginate lyases and determination of substrate specificity. Appl and Environ Microbiol 56: 2265-2267 [12].

Des aliquotes de 2 microlitres des fractions de protéine sont déposées sur des gels d'agarose (1% en poids d'agarose, 0,1% en poids d'ulvanes, 20mM Tris-pH 7,7 ou 8,5, 200mM NaCl). La boîte de pétrie a été maintenue durant la nuit à la température ambiante, ici 20°C, et la présence d'activité enzymatique est révélée par l'ajout d'une solution de rouge de ruthénium (0,5% en poids dans de l'eau) pendant 10 minutes ou pendant 10- à 30 minutes. Les fractions actives sont identifiées par des tâches claires (non colorées) sur fond rose.

Les protéines purifiées ont migrées sur gel d'électrophorèse puis ont été colorés par du bleu colloïdal. Les bandes de protéine ont été excisées des gels de SDS-PAGE et ont été analysées par spectrométrie de masse sur la plateforme RIO « Bioploymères » INRA, à Nantes.

Les inventeurs ont ainsi isolé une protéine de 46 kD de séquence SEQ IDn°4.

Les séquences des peptides obtenus après incubation à la trypsine n'avaient aucune homologie significative avec des séquences de l'art antérieur dans la banque TrEMBL malgré la taille importante des fragments peptidiques séquencés.

Les protéines, dites protéines ulvanolytiques, de 30kD isolée dans l'exemple 3 et de 46 kD isolée dans le présent exemple ont été purifiées sur gel d'électrophorèse comme représenté sur la figure 1 annexée : gel d'électrophorèse de la protéine de 30kD (Gel A) et de la protéine 46 kD (Gel B). Les flèches indiquent les bandes qui ont été excisées puis utilisées pour le séquençage des peptides par spectrométrie de masse.

**Tableau 1 : Séquences peptidiques obtenues à partir des protéines ulvanolytiques de 30 kD et 46 kD par séquençage de novo**

| **Masse moléculaire** | | | |
|---|---|---|---|
| **30 kD** | | **46 kD** | |
| **Séquences** | **SEQ ID NO.** | **Séquences** | **SEQ ID NO.** |
| **PNDPNLK** | 9 | **PNDPNLK** | 9 |
| B**ILEVGNTGTFGSTGS**A | 13 | I**LEVGNTGTFGSTGS**YLMQAK | 30 |
| **DLANPDNV** | 11 | **DLANPDNV**GTVDDR | 31 |
| **ALL**GG**Q**VFN**WNIPE**S | 14 | QEM**ALL**M**Q**EVD**WNIPE** | 32 |
| BEQLNFR | 15 | ------------------ADLYR | 33 |
| LELLDLELE | 16 | WDNSTLPAADLYR | 34 |
| TGVGSYAR | 17 | ADLYR | 35 |
| BPVYG-NQVQVSFDLWR | 18 | YHDTNNMLTHSANLDDR | 36 |
| GGGGSNDPALCLYLAR | 19 | LYENGELVDEFL | 37 |
| ACPSSGVFQ | 20 | | |
| LLSGWG | 21 | | |
| BVYDNTLV | 22 | | |
| FGVTGPPT | 23 | | |
| DLLGNTLD | 24 | | |
| BDTDLPNPR | 25 | | |
| ATGAG | 26 | | |
| BSCYANYSESSLLGK | 27 | | |
| BDDPNNPGQTLHYAWK | 28 | | |
| BFWGLYNLTD | 29 | | |

Les séquences en gras correspondent aux séquences communes aux ulvanes lyases de 30 et 46 kD de la présente invention. Les quatre premiers peptides sont communs pour les deux protéines. Ces données confrontées à la banque TrEMBL n'ont pas permise d'identifier de séquence homologue comprenant ces quatre séquences dans une seule protéine.

Les séquences soulignées ont été employés pour la construction des amorces dégénérées utilisées dans l'exemple suivant.

### Exemple 5 : identification d'un gène codant

### 5.1) Amorces dégénérées

Six amorces dégénérées ont été synthétisées sur la base des séquences peptidiques communes aux protéines de 30 et 46kD obtenues à partir de la spectrométrie de masse dans les deux directions (Forward : F et Reverse : R; tableau 2).

Une amplification par PCR a été réalisée avec toutes les combinaisons possibles des amorces sur 75 ng d'ADN génomique de 01-PN-2010 dans des milieux réactionnels de 25 µL contenant tampon GoTaq PCR 1x, 1 mM MgCl₂, 0.2 mM de chaque dNTP, 2 µM des amorces Forward et Reverse, et 1,25 U GoTaq (Promega). Le programme d'amplification était de 94°C pendant 2 minutes, trente-cinq cycles de : 94°C pendant 30 sec, 50°C pendant 30 sec et 72°C pendant 1 min 30 sec suivi par une étape finale à 72°C pendant 7 minutes. Ensuite les échantillons ont été maintenus à 4°C avant séquençage.

De cette façon, les inventeurs ont obtenu un fragment de 700 pb du gène d'ulvane lyase

### 5.2) « Tail PCR »

La méthode de TAIL-PCR décrite dans le document Liu YG and Whittier RF 1995 Thermal Asymmetric Interlaced PCR: Automatable Amplification and Sequencing of Insert End Fragments from P1 and YAC Clones for Chromosome walking. Genomics 25: 674-681 [13] a été utilisée pour obtenir la séquence des extrémités du gène d'ulvane lyase.

Les amorces « spécifiques » sont des amorces dégénérées conçues en se basant sur les fragments de séquence du gène connue (Tableau 2). Cinq amorces dégénérées « arbitraires » différentes ont été choisies parmi celles déterminées dans la littérature (tableau 2 dans le document Liu et Whittier et al [6], et dans le document Liu YG, Mitsukawa N, Oosumi T and Whittier RF 1995 Efficient isolation and mapping of Arabidopsis thaliana T-DNA insert junctions by thermal asymmetric interlaced PCR. Plant J. 8: 457-463 [14]).

Les programmes d'amplification étaient différents pour les différentes réactions de TAIL PCR (Tableau 3) et sont basés sur les programmes de Liu et al 1995 [14] mais adaptés aux machines de PCR disponibles au laboratoire.

**Tableau 2 : Amorces utilisées pour identifier le gène de l'ulvane lyase (F : forward, R : reverse)**

| Amorce | SEQ ID NO. | Séquence (5' à 3') | SEQ ID NO. | Tₘ |
|---|---|---|---|---|
| **Amorces dégénérées de l'ulvane lyase** | | | | |
| VGNTGTFG-F | 64 | GTN GGN AAY ACN GGN ACN TTY GG | 38 | 52-64 |
| PNDPNLK-F | 9 | CCN AAY GAY CCN AAY YTN AA | 39 | 42-56 |
| ANPDNVG-F | 65 | GCN AAY CCN GAY AAY GTN GG | 40 | 48-60 |
| TLPAADLY-F | 66 | ACN YTN CCN GCN GCN GAY YTN TA | 41 | 50-66 |
| GGQVFNW-F | 67 | GGN GGN CAR GTN TTY AAY TGG | 42 | 49-60 |
| GQTLHYAW-F | 68 | GGN CAR ACN YTN CAY TAY GCN TGG | 43 | 52-66 |
| VGNTGTFG-R | 64 | CCR AAN GTN CCN GTR TTN CCN AC | 44 | 52-64 |
| PNDPNLK-R | 9 | TTN ARR TTN GGR TCR TTN GG | 45 | 42-56 |
| ANPDNVG-R | 65 | CCN ACR TTR TCN GGR TTN GC | 46 | 48-60 |
| TLPAADLY-R | 66 | TAN ARR TCN GCN GCN GGN ARN GT | 47 | 50-66 |
| GGQVFNW-R | 67 | CCA RTT RAA NAC YTG NCC NCC | 48 | 49-60 |
| GQTLHYAW-R | 68 | CCA NGC RTA RTG NAR NGT YTG NCC | 49 | 52-66 |

| **Amorces spécifiques pour TAIL PCR** | | | | |
|---|---|---|---|---|
| UL_133R | - | CTAG GTT GTA ATG TGT TAG GTG CAT CCC | 50 | 60 |
| UL_194R | - | GTG AAT CGC GCA TAA CTT CCC ACA CC | 51 | 61 |
| UL_285R | - | CC CGT GTG CTT ACC TTT GGC CTG C | 52 | 63 |
| UL_426F | - | GC AGC TGG AAG AAC CGA GGT CTT TC | 53 | 61 |
| UL_582F | - | CCG GAA CCA GAA CGA GGA AGA GAA TC | 54 | 61 |
| UL_643F | - | GGA GGA AGA GCA CAA ATG AGA TGG GC | 55 | 61 |
| AfterUL 1F | - | | 56 | 61 |
| AfterUL 2F | - | GCT TCT GTA GGT GTG TAT CCT AAC CC | 57 | 60 |
| AfterUL 3F | - | GCT GGA CGT GTG TCT TCT TTG TAT TAC GC | 58 | 62 |

| **Amorces arbitraires dégénérées pour TAIL PCR** | | | | |
|---|---|---|---|---|
| AD1 | - | TGW GNA GWA NCA SAG A | 59 | 38-43 |
| AD2 | - | AGW GNA GWA NCA WAG G | 60 | 38-43 |
| AD3 | - | WGT GNA GWA NCA NAG A | 61 | 38-43 |
| AD4 | - | NTC GAS TWT SGW GTT | 62 | 36-39 |
| AD5 | - | NGT CGA SWG ANA WGA A | 63 | 38-43 |

| **Amorces arbitraires pour clonage dans pFO4** | | | | |
|---|---|---|---|---|
| UL_BgIII_F | - | | 69 | - |
| UL EcoRI_R | - | | 70 | - |

**Tableau 3 : Conditions d'amplification utilisées pour le Tail-PCR**

| **Réaction** | **Nombre de cycles** | **Températures et durées** |
|---|---|---|
| Primaire | 1 | 93°C, 2 min |
| | 5 | 94°C, 1 min; 62°C, 1 min; 72°C, 2 min |
| | 2 | 94°C 1 min; ramping to 25°C over 3 min; 25°C, 3 min; ramping to 72°C over 3 min; 72°C, 2 min |
| | 15 | 94°C, 30 sec; 65°C, 1 min; 72°C, 2 min; 94°C, 30 sec; 65°C, 1 min; 72°C, 2 min; 94°C, 30 sec; 45°C, 1 min; 72°C, 2 min |
| | 1 | 72°C, 7 min; 4°C, ∞ |
| Secondaire | 1 | 93°C, 1 min |
| | 13 | 94°C, 30 sec; 62°C, 1 min; 72°C, 2 min; 94°C, 30 sec; 62°C, 1 min; 72°C, 2 min; 94°C, 30 sec; 45°C, 1 min; 72°C, 2 min |
| | 1 | 72°C, 7 min; 4°C, ∞ |
| Tertiaire | 1 | 93°C, 1 min |
| | 20 | 94°C, 30 sec; 45°C, 1 min; 72°C, 2 min |
| | 1 | 72°C, 7 min; 4°C, ∞ |

### Exemple 6 : Protocole de digestion d'ulvanes par les ulvanes lyases de la présente invention

25 µL d'une fraction d'ulvane lyase pure avec une concentration de 5,7 µg protéine par mL était ajouté dans 1 mL milieu réactionnel compose de NaCl (200 mM), ulvane (1 g L-1) et Tris HCl (20 mM, pH 9,2) à 35 °C dans une cuvette de quartz. La dégradation de l'ulvane (ou plutôt la formation de double liaison) était suivi par l'augmentation de l'absorbance à 235 nm.

### Exemple 7 : dégradation d'oligo-ulvanes par les ulvanes lyases de la présente invention

La figure 4 représente les résultats obtenus d'expériences de chromatographie échangeuse d'ions conduites avant ou après incubation d'un disaccharide (graphique A) ou d'un tétrasaccharide (graphique B) avec les ulvanes lyases de la présente invention obtenues dans les exemples ci-dessus, dans les mêmes conditions que l'exemple précédent.

La dégradation du tétrasaccharide démontre l'activité glucuronique lyase de l'enzyme. Sur ces graphiques, l'axe des abscisses représente temps d'élution en minutes (min), et l'axe des ordonnées représente conductométrie en nano-Coulomb (nC).

### Exemple 8 : expression hétérologue et dégradation d'oligo-ulvanes par les ulvanes lyases de la présente invention

Le module catalytique du gène de l'ulvane lyase identifié dans l'exemple 5 précité a été amplifié avec des amorces spécifiques permettant l'incorporation des sites de restriction BgIII et EcoRI aux extrémités 5' et 3' du fragment, respectivement (Tableau 2).

Des conditions standard de PCR, à savoir 1x GoTaq PCR buffer, 1 mM MgCl₂, 0,2 mM de chaque dNTP, 2 µM des amorces « sens » forward et « anti-sens » reverse, 1,25 U GoTaq (Promega) et 15 ng ADN génomique, ont été utilisées avec une température d'hybridation de 50°C et 30 cycles de polymérisation. Les produits PCR ont été ensuite purifiés, digérés par les enzymes de restrictions appropriés i.e. *Bg*/II et EcoRI et sous clonés dans le vecteur d'expression pFO4 modifié de pET15 (Novagen) pour être compatible avec les stratégies de ligation *Bam*HI*lEco*RI and *Bgl*II/*Mfe*I*.*

Les plasmides recombinants ont été utilisés pour transformer la souche BL21 (DE3) *d'Escherichia coli* préparés au laboratoire selon le protocole de Cohen, SN, Chang ACY, Hsu L (1972) Nonchromosomal antibiotic resistance in bacteria: genetic transformation of Escherichia coli par R-factor DNA. Proc. Natl. Acad. Sci. USA 69: 2110-2114 [15]. Des colonies transformées ont été cultivées d'abord 3 h à 37°C dans un milieu d'expression à base de Luria-Bertani (10 g tryptone, 5 g extrait de levure et 10 g NaCl per L) avec ampicilline et 0,5 % glucose. Ensuite un volume égale de milieu Luria-Bertani froid avec 0,6% lactose, 20 mM Hepes pH 7,0 et 1 mM d'isopropyl β-D-1-thiogalactopyranoside (IPTG) a été ajouté et la culture incubée à 20°C pendant 18h.

Après centrifugation le culot bactérien a été suspendu dans un tampon 20 mM Tris-HCl, 500 mM NaCl et 5 mM imidazole avec un pH de 7,4. La lyse de cellules a été réalisée dans une presse de French et les débris bactérien enlevé par centrifugation à 20000 tpm. Le surnageant a été appliqué sur une colonne de Ni Sepharose chargé de 100 mM NiSO₄ (GE Healthcare). Après lavage les protéines attachées ont été éluées par un gradient linéaire d'imidazole de 5 mM à 500 mM. Les fractions actives ont été regroupées et dessalées sur une colonne HiPrep 26/10 de dessalage à l'équilibre dans 20 mM Tris-HCl pH 8,0.

Les fractions actives ont été identifiées par le test en boîte de Pétri, décrit dans exemple 4.

L'expression hétérologue du module catalytique de l'ulvane lyase a montrée une dégradation d'ulvanes sur les boites de Pétri, taches claires sur la figure 5 confirmant ainsi la fonction de catalytique.

### 2. Chromatographie haute performance échangeuse d'anions (HPAEC)

La pureté des fractions d'oligosaccharides et la cinétique de dégradation d'oligosaccharides pures a été analyse par HPAEC avec un appareil de chromatogarphie Dionex ICS 3000 équipé d'une boucle d'injection de 20 µL, un système d'injection automatique AS100XR (Thermo Separation Products) et d'une colonne échangeuse d'anions AS11 anion (4 mm x 250 mm, Dionex IonPac) associée à une colonne de garde AG11 (4 mm x 50 mm, Dionex IonPac). Le système a été mis en oeuvre dans un mode conductivité utilisant un détecteur ED40 (Dionex) et un suppresseur Dionex ASRS ultra-4mm avec un courant de 300 mA. Les phases mobiles étaient de l'eau ultrapurifiées et 290 mM de NaOH. L'élution a été réalisée à un débit de 0,5 mL min⁻¹ avec une pompe gradient GP40. Le gradient utilisé était 0 min, 3%B; 1,5 min 1%B; 4,1 min 5%B; 6,5min 10%B; 10,0 min 18%B; 26 min 22% B; 28 min 40%B; 30 min 100%B; 30,1 min 3%B; 37 min 3% B. Le programme Chromeleon-peak Net software (Dionex) a été utilisé pour l'acquisition des donnés et le transfert. Les di- et tétrasaccharides ont été incubés avec les ulvanes lyases purifiées de l'invention et analyses par HPAEC.

Le Δ-R3S disaccharide, c'est à dire (acide 4-deoxy-L-threo-hex-4-enopyranosiduronic lié en 4 au L-Rhap 3-sulfate) Figure 6 A et le tétrasaccharide Δ -R3S-Xyl-R3S n'ont pas été modifié par les enzymes et sont des produits finaux. Le mélange de Δ-R3S-GlcA-R3S et Δ-R3S-IduA-R3S tétrasaccharides (70:30, 50:50 et 30:70, GlcA:IduA ratios) ont été entièrement convertis dans un seul disaccharide: Δ-R3S, montrant que l'ulvan-lyase a clivé la liaison glycosidique entre le rhamnose sulfaté et soit le résidu glucuronique ou iduronique. Les figures 6 B et C représentent les résultats obtenus montrant les pics de chaque saccharide. La cinétique de dégradation de ces mélanges a été suivie par HPAEC afin de mettre en avant les possibles différences de reconnaissance des résidus glucuronique ou iduronique.

Toutefois, la vitesse de dégradation des mélanges de tétrasaccharides et la vitesse de production du disaccharide Δ-R3S étaient indépendantes du ratio GlcA:IduA. Les vitesses de dégradation de 0,18±0,05 µM/min et 0,08±0,02 ont été observées pour les ulvanes lyases de 30 kD et 46 kD. Les dégradations ont été réalisées avec des concentrations de tétrasaccharides 1,5mM dans 200 mM de carbonate d'ammonium à 30° C.

Tel que démontré dans cet exemple, les ulvanes lyases de l'invention ou décrites permettent donc une coupure au niveau de l'acide glucuronique et également au niveau de l'acide iduronique, démontrant que l'ulvane lyase de la présente invention ont une activité différente et supérieure de celles de l'art antérieur.

### Listes des références

[1] Marc Lahaye et Audrey Robic, Structure and functional properties of ulvan, a polysaccharide from green seaweeds. Biomacromolecules 2007, Vol. 8, 1765-1774.
[2] http://www.cbs.dtu.dk/services/SignalP/.
[3] http://bmbpcu36.leeds.ac.uk/prot analysis/Signal.htmL.
[4] http://www.promega.com/vectors/mammalian_express_vectors.htm.
[5] http://www.qiagen.com/overview/qiagenes.aspx?gaw=PROTQIAgene s0807&gkw=mammalian+expression.
[6] http://www.scbt.com/chap_exp_vectors.php?type=pCruzTM%20Expr ession%20Vectors.
[7] WO 83/004261.
[8] ZoBell, CE 1941 Studies on marine bacteria. I. The cultural requirements of heterotrophic aerobes, J Mar Res 4, 41-75.
[9] Lahaye M., Bimalendu R., Baumberger S., Quernener B. and Axelos M., Procédé d'extraction des ulvanes, (1996) Hydrobiologia, 326/327, 473.
[10] LaemmLi UK and Favre M 1973 Links maturation of the head of bacteriophage T4.I. DNA packaging events. J Mol Biol 80:575-599.
[11] Bradford MM 1976 ; A rapid and sensitive method for the quantitation of microgram quantities of protein using the principle of protein-dye binding. Anal Biochem 72: 248-254.
[12] Gacesa P and Wusteman FS 1990 Plate assay for simultaneous detection of alginate lyases and determination of substrate specificity. Appl and Environ Microbiol 56: 2265-2267.
[13] Liu YG and Whittier RF (1995), Thermal Asymmetric Interlaced PCR : Automatable Amplification and Sequencing of Insert End Fragments from P1 and YAC Clones for Chromosome walking. Genomics 25: 674-681.
[14] Liu YG, Mitsukawa N, Oosumi T and Whittier RF (1995), Efficient isolation and mapping of Arabidopsis thaliana T-DNA insert junctions by thermal asymmetric interlaced PCR. Plant J. 8: 457-463.
[15] Cohen, SN, Chang ACY, Hsu L (1972) Nonchromosomal antibiotic resistance in bacteria: genetic transformation of Escherichia coli by R-factor DNA. Proc. Natl. Acad. Sci. USA 69: 2110-2114

## Revendications

1. Ulvane lyase ayant une activité iduronique et glucuronique lyase extraite du microorganisme déposé le 17 juin 2010 sous le numéro I-4324 à la collection nationale de cultures de micro-organismes (CNCM) 25 rue du docteur Roux, 75724 Paris Cedex 15, France, de 46kD de séquence SEQ ID NO 4.

2. Acide nucléique de séquence SEQ ID n°8.

3. Vecteur comprenant un acide nucléique selon la revendication 2.

4. Cellule hôte comprenant une séquence d'acide nucléique selon la revendication 2 ou un vecteur selon la revendication 3.

5. Microorganisme déposé le 17 juin 2010 sous le numéro I-4324 à la collection nationale de cultures de micro-organismes (CNCM) 25 rue du docteur Roux, 75724 Paris Cedex 15, France.

6. Procédé de fabrication d'une ulvane lyase selon la revendication 1 par recombinaison génétique utilisant un acide nucléique selon la revendication 2 ou un vecteur selon la revendication 3.

7. Procédé de dégradation d'ulvanes comprenant une étape de mise en contact des ulvanes avec une ulvane lyase selon la revendication 1 ou avec une cellule hôte selon la revendication 4 ou avec un microorganisme selon la revendication 5, dans les conditions permettant la dégradation des ulvanes par digestion enzymatique par ladite protéine ou ledit hôte ou ledit microorganisme.

## Patentansprüche

1. Ulvanlyase mit iduronischer und glucoronischer Lyaseaktivität aus einem am 17. Juni 2010 in der Collection nationale de cultures de mirco-organismes, CNCM (Nationale Sammlung von Kulturen von Mikroorganismen), 25 rue du docteur Roux, 75724 Paris Cedex 15, Frankreich, mit der Nummer I-4324 angemeldeten Mikroorganismus von 46kD mit der Sequenz SEQ ID NO 4.

2. Nukleinsäure der Sequenz SEQ ID n°8.

3. Vektor umfassend eine Nukleinsäure nach Anspruch 2.

4. Wirtszelle umfassend eine Nukleinsäuresequenz nach Anspruch 2 oder einen Vektor nach Anspruch 3.

5. Ein am 17. Juni 2010 in der Collection nationale de cultures de mirco-organismes, CNCM (Nationale Sammlung von Kulturen von Mikroorganismen), 25 rue du docteur Roux, 75724 Paris Cedex 15, Frankreich, mit der Nummer I-4324 angemeldeter Mikroorganismus.

6. Verfahren zur Herstellung einer Ulvanlyase nach Anspruch 1 durch genetische Rekombination unter Verwendung einer Nukleinsäure nach Anspruch 2 oder eines Vektors nach Anspruch 3.

7. Abbauprozess von Ulvanen umfassend einen Schritt des Inkontaktbringens der Ulvane mit einer Ulvanlyase nach Anspruch 1 oder mit einer Wirtszelle nach Anspruch 4 oder mit einem Mikroorganismus nach Anspruch 5 unter Bedingungen, die den Abbau der Ulvane durch enzymatische Verdauung des Proteins oder der Wirtszelle oder des Mikroorganismus ermöglichen.

## Claims

1. Ulvan lyase having an iduronic and glucuronique lyase activity extracted from the microorganism deposited on 17 June 2010 under number I-4324 in the National Collection of Cultures of Microorganisms (CNCM) 25 rue du docteur Roux, 75724 Paris Cedex 15, France, of 46kD of sequence SEQ ID NO 4.

2. Nucleic acid of sequence SEQ ID No.8 in the appended sequence listing.

3. Vector comprising a nucleic acid according to Claim 2.

4. Host cell comprising a nucleic acid sequence according to Claim 2 or a vector according to Claim 3.

5. Microorganism deposited on 17 June 2010 under number I-4324 in the National Collection of Cultures of Microorganisms (CNCM) 25 rue du docteur Roux, 75724 Paris Cedex 15, France.

6. Method of manufacturing an ulvan lyase according to Claim 1 by genetic recombination using a nucleic acid according to Claim 2 or a vector according to Claim 3.

7. Method of degrading ulvans comprising a step of bringing the ulvans into contact with an ulvan lyase according to Claim 1 or with a host cell according to Claim 4 or with a microorganism according to Claim 5, in conditions allowing degradation of the ulvans by enzymatic digestion by said protein or said host or said microorganism.
